# EUROPEAN PATENT APPLICATION

(11) **EP 4 721 765 A1**
(43) Date of publication of application: **08.04.2026**
(21) Application number: 24306633.9
(22) Date of filing: 04.10.2024
(51) Int. Cl.: A61K 47/68, A61P 35/00, C07K 16/28

(54) **BI-FUNCTIONALIZED ANTIBODY CONJUGATES AGAINST THE ENDOTHELIN RECEPTOR B**

(71) Applicant: Commissariat à l'Energie Atomique et aux Energies Alternatives, 75015 Paris (FR); Université de Bourgogne, 21078 Dijon (FR); Université Paris-Saclay, 91190 Gif-sur-Yvette (FR); Centre National de la Recherche Scientifique, 75016 Paris (FR); Institut National de la Recherche pour l'Agriculture, l'Alimentation et l'Environnement, 75007 Paris (FR)
(72) Inventor: BOQUET, Didier, 91191 Gif-sur-Yvette cedex (FR); HERBET, Amaury, 91191 Gif-sur-Yvette cedex (FR); COSTA, Narciso, 91191 Gif-sur-Yvette cedex (FR); HAUTIERE, Marie, 91191 Gif-sur-Yvette cedex (FR); DENAT, Franck, 21078 Dijon (FR); VIVIER, Delphine, 21078 DIJON (FR)
(74) Representative: Santarelli

(57) **Abstract**

The present invention relates to novel antibody conjugates containing chimeric or humanized antibodies (or fragments thereof) that are able to recognize specifically the human Endothelin B receptors expressed on tumor cells, said antibodies or fragments carrying at least two functional molecules that are cytotoxic and / or detectable. The invention also relates to the use of such bifunctionalized antibody conjugates as diagnostic agents and/or as medicaments, for detecting and/or treating patients suffering from melanoma and other cancers in which the human endothelin B receptor is overexpressed, such as glioblastoma, bladder cancer, lung cancer, kidney cancer, or vulvar cancer.

## Description

### SUMMARY OF THE INVENTION

The present invention relates to novel antibody conjugates containing chimeric or humanized antibodies (or fragments thereof) that are able to recognize specifically the human Endothelin B receptors expressed on tumor cells, said antibodies or fragments carrying at least two functional molecules that are cytotoxic and / or detectable. The invention also relates to the use of such bi-functionalized antibody conjugates as diagnostic agents and/or as medicaments, for detecting and/or treating patients suffering from melanoma and other cancers in which the human endothelin B receptor is overexpressed, such as glioblastoma, bladder cancer, lung cancer, kidney cancer, or vulvar cancer.

### BACKGROUND OF THE INVENTION

Endothelins (ETs) constitute a family of 3 21-amino acid peptides, ET-1, ET-2 and ET-3, which bind to two distinct 7-transmembrane domain receptors: ET_{A} (standing for "Endothelin subtype A receptor") and ET_{B} (standing for "Endothelin subtype B receptor"), these two receptors belonging to the G protein-coupled receptor (GPCR) family. The endothelin axis (endothelins and their receptors) is strongly involved in physiological and pathological processes. ET-1 plays a crucial role in the regulation of physiological smooth muscle motility, but ET-1 is also implicated in a large variety of pathologies, including hypertension, heart failure, kidney disorders and infectious diseases. In addition, the ET axis is overexpressed in cancer of different organs contributing to tumor growth by acting on cell proliferation, survival, migration, differentiation, angiogenesis and inflammatory cell recruitment. ET_{A} are upregulated in prostate, ovary and breast cancers while ET_{B} is deregulated in melanoma, lung, renal and vulvar cancers (Rosano et al, 2013).

Melanoma is an aggressive cancer that presents an increased incidence rate. This cancer is characterized by its capacity to metastasize promptly, leading to an increase in mortality rates in many countries. Somatic mutations have been found in BRAF and N-RAS genes in about 50% and 20% of melanomas, respectively, resulting in constitutive activation of ERK1/2 MAPK pathway. Moreover, gene expression profiling and targeted approaches have demonstrated that ET_{B} expression is upregulated in melanoma. The upregulation of ET_{B} is involved in proliferation, migration and angiogenesis associated with tumor growth and invasiveness. In melanoma, ET-1 via ET_{B} expressed on cancer cells modulates migration and formation of vasculogenic mimicry via the upregulation of HIF/ VEGF/VEGFR pathway. These data implicate ET_{B} as a potential driver of melanoma progression and an important marker of aggressive phenotype. Hence new therapeutic molecules targeting ET_{B} need to be developed in order to inhibit the ET pleiotropic effects on melanoma cells.

An ET_{B}-specific peptidic antagonist (BQ788) has been used in basic research to reduce the proliferation of cancer cells. Preclinical trial confirmed the efficacy of BQ788 on melanoma growth (Lahav R. 2005) but with poor clinical interest (Wouters J. et al, 2015).

The dual receptor antagonist bosentan was also successfully used in melanoma cell lines (Berger Y et al., 2006), yet the two Phase 2 studies in patients with stage IV metastatic melanoma were disappointing (Maguire JJ et al, 2015; Kefford RF et al, 2010). Used as a monotherapy, bosentan stabilized less than 20% of the patients, and no additional effect on temporal progression of tumors was observed when bosentan was combined with a chemotherapeutic agent (dacarbazine). Macitentan was recently tested for the treatment of recurrent glioblastoma, but the results show little effect on the tumoral progression (Weathers SP et al, 2021).

In this context, the development of other therapeutic molecules targeting ET_{B} is needed to block the upregulated signaling pathways that occur in melanoma upon ET induction.

The development of monoclonal antibodies (mAbs) for the treatment of cancers is an alternative to conventional therapeutic agents and is currently a rapidly expanding sector. In the particular case of metastatic melanoma treatment, the anti-CTLA-4 blocking antibody ipilimumab and the anti-PD-1 antibodies pembrolizumab and nivolumab that modulate the immune checkpoints have been recently approved (Russo A. et al, 2014; Turneh PC et al, 2014; Topalian SL et al, 2014). However, despite the improvement of overall survival observed with these mAbs, they do not lead to remission of the disease.

Compared to small pharmacological molecules, mAbs can detect fine antigenic differences between normal and pathologic cells, inhibiting different functions involved in cell growth, migration, angiogenesis or metastasis. Moreover, mAbs display various cytotoxic actions through the immune system, and they can be coupled to several imaging tracers and markers or cytotoxic molecules. For example, the mAb Trastuzumab, which is directed against the human epidermal growth factor receptor HER-2 often overexpressed in breast cancer, has been shown to significantly improve the overall survival of HER2-positive cancer patients (Recondo G. et al, 2014).

Like HER-2 in breast cancer, the ET_{B} receptor is overexpressed at the surface of melanoma cells and it is therefore tempting to try to antagonize this receptor with mAbs. Yet the ET_{B} receptor has a complex three-dimensional structure, and it is difficult to obtain a native and functional form of the polypeptide, outside its membrane context. Moreover, ET_{B} as ET_{A} are allosteric receptors, i.e. capable to have conformational modifications after the ligand binding (Shihoya et al. 2016). Several mAbs have been nevertheless proposed to target ET_{B}:
Kondoh et al, 1990 ("Isolation of anti-endothelin receptor monoclonal antibodies for use in receptor characterization", BBRC, vol. 172, pages 503-510) describe the binding properties of 4 monoclonal antibodies (A2, G9, E7 and G10) to solubilized complexes of endothelin receptors. Yet, these authors do not provide any information about the fine specificity of these antibodies (against the receptors ET_{A} and/or ET_{B}), as regards the recognition of human origin receptors, nor as regards a possible antagonistic property.

Yamaguchi et al, 2004 ("Characterization and application of monoclonal antibodies against human endothelin B receptor expressed in insect cells", Biotechnology Letters, vol. 26, pages 293-299) showed the binding properties of 5 mouse monoclonal antibodies obtained after protein immunization with recombinant human ET_{B} produced in insect cells. Four of them had a similar affinity (in the nanomolar range) for ET_{B}, whereas the fifth one was 10 times less affine. The epitope analysis of 3 of them (N-6, N-3 and N-1) revealed that they recognized the ET_{B} N-terminal domain and more particularly the sequence corresponding to amino acids 27-35 of the ET_{B} (for N-6); the sequence corresponding to amino acids 27-41 of the ET_{B} (for N-3) and the sequence corresponding to amino acids 71-85 (for N-1). Finally, these three antibodies were capable of recognizing COS cells over-expressing human ET_{B}.

Patent application JP 2012111706 related to a monoclonal antibody called hB07. This was an IgG2a/lambda isotype mouse immunoglobulin, which was specific to human ET_{B}. hB07 was capable of competitively blocking endothelin 1 (ET-1) binding to ET_{B} with an efficiency (IC₅₀) calculated of 1.7 10⁻⁷M.

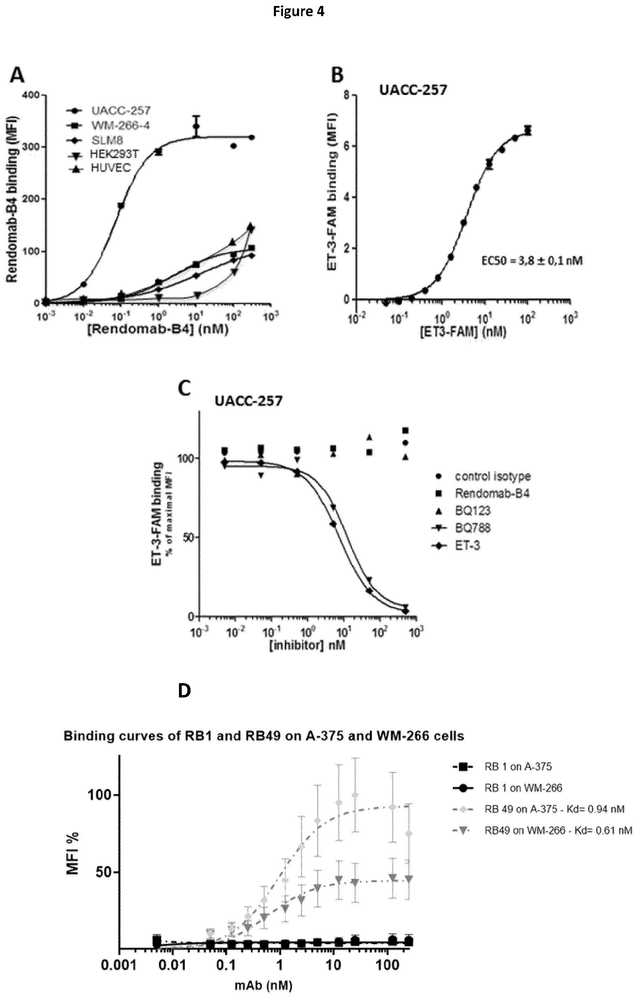

Another monoclonal antibody antagonist to human ET_{B}, called Rendomab-B1, has been described in (Allard B. et al, 2013). Rendomab-B1 was the first-reported mAb behaving as a potent antagonist of human ET_{B}, blocking ET-1-induced signaling in CHO cells overexpressing ET_{B}. Nevertheless, rendomab B1 displayed a very low binding for ET_{B} overexpressed at the surface of some human melanoma cells (for example: A375; WM-266-4), suggesting either a structural heterogeneity among ET_{B} receptors expressed by different cell types, or a high level of ET_{B} receptors complexed with endothelin ligand preventing the rendomab B1 binding (Figure 4D).

After that, a therapeutic antibody called 5E9 has been obtained, targeting the human ET_{B} over-expressed at the surface of melanomas (WO 2013/063001). This antibody 5E9 however cross-reacted with the rodent ET_{B} as well as with the non-human primate receptor (Asundi J. et al, 2011). Moreover, it had no inhibitory effect by itself. It was nevertheless conjugated with monomethyl auristatin E, and showed good efficacy against human melanoma cell lines and xenograft tumor models.

Finally, three antibodies specific of the subtype B of the human endothelin receptor ("rendomab-B49", "rendomab-B41", and "Rendomab-B36") were generated (WO2017/220739). These antibodies recognized the human ET_{B} receptor expressed by glioblastoma cells with a strong affinity, but they were not able to antagonize efficiently this receptor; i.e., they were not capable of inhibiting or blocking the binding of the endothelin ligands (ET1, ET2 or ET3) on same.

Altogether, it appears from the analysis of all these prior art documents that there is still a need of identifying an antibody recognizing i) the epitopes of SEQ ID NO:9 and SEQ ID NO:10 on human ET_{B} receptor specifically, ii) when said receptors are expressed at the surface of human melanoma cells, iii) with very high affinity and iv) capable to inhibit the PLC signaling pathway and melanoma migration. The present invention solves this need, by providing a novel anti-human ETB mAb (clone Rendomab-B4, hereafter referred to as the "antibody of the invention"), that has been selected on its capacity to recognize specifically the human ET_{B} receptor at the surface of melanoma cells, and that efficiently inhibits the ET_{B} dependent G-protein signaling and migration of human melanoma cells.

Another technical problem solved by the present invention was to provide an efficient antibody conjugate that would keep the above-mentioned advantageous anti-tumor characteristics and would be tagged or coupled to at least two different kinds of functional molecules (either two different imaging probes, or two different cytotoxic compounds, or one of each category). To solve this need, the present inventors propose using a targeted double labelling which is easy to implement, regioselective, biocompatible and modular, using a trivalent linking chemical entity (or "trifunctional platform") allowing the regioselective binding of at least two different kinds of labels or drugs to the antibody of the invention (or to a fragment thereof).

The present invention therefore provides bi-functionalized antibody conjugates (ACs) comprising the variable domain of the antibody of the invention and at least two different kinds of functional molecules (either two different imaging probes, or two different cytotoxic compounds, or one of each category) that can be used to detect, diagnose and/or treat solid cancers in which the human endothelin B receptor is overexpressed, such as melanoma, glioblastoma, pancreatic cancer, ovarian cancer, bladder cancer, lung cancer, kidney cancer, or vulvar cancer.

### DETAILED DESCRIPTION OF THE INVENTION

### Description of RB4

The present inventors herein report the identification of a novel anti-human ET_{B} mAb (clone Rendomab-B4, hereafter referred to as the "antibody of the invention"), that has been selected on its capacity to recognize specifically the human ET_{B} receptor (herein called ET_{B}) at the surface of melanoma cells. The Complementary Determining Regions (CDRs) of this mAb were obtained by cloning and sequencing. These CDRs can be used to generate a mouse-human chimeric antibody, a humanized or a fully human antibody that will be able to treat human patients suffering from a melanoma cancer, or other solid or liquid cancers, after their functionalization as described below.

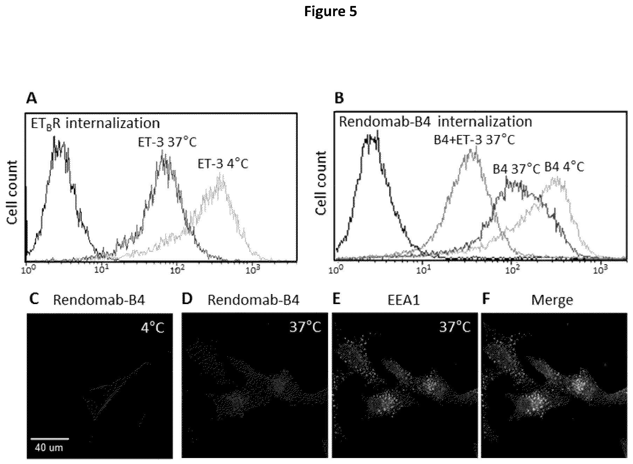

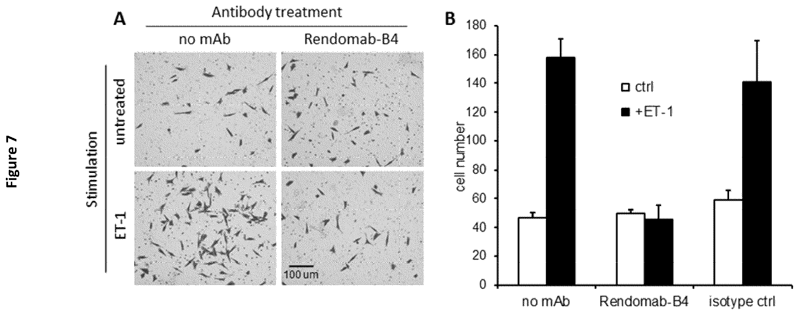

To obtain the antibody RB4, the inventors have used a particular selection immunisation strategy, coupled with a hybridoma screening procedure in ELISA-cell and then by flow cytometry that favours the obtention of monoclonal antibodies specific to ET_{B} in its native conformation (Allard B. et al, 2013). By using this genetic immunization, they identified that RB4 advantageously :
- is able to recognize UACC-257 melanoma ET_{B} with very high affinity subnanomolar (0.15 nM (figures 1 and 2), and to inhibit the phospholipase C activation, cf. figure 6A) from this receptor,
- does not recognize the other sub-type of endothelin receptor ET_{A} (not shown),
- does not inhibit ERK1/2 pathway nor the β-arrestin-dependent signaling in melanoma cells,
- recognizes the epitopes of SEQ ID NO:9 and SEQ ID NO:10 on ET_{B} R,
- promotes the ET_{B} internalisation in melanoma cells (figure 5),
- completely inhibit the migration of melanoma cells induced by ET-1 (figure 7).

Importantly, the RB4 antibody is not capable of recognizing or inhibiting the other endothelin sub-type receptor ET_{A}, nor the ET_{B} receptor from murine species. Its effect is therefore opposite to the other ET_{B} antibodies of the prior art.

The RB4 antibody is the first reported mAb to possess the ability to differentially affect ET_{B}-coupled signaling pathways, behaving as a biased allosteric modulator inhibiting G protein-dependent processes (e.g., PLC activation) but not ERK1/2 and β-arrestin-dependent ones.

Importantly, the inventors herein showed (figure 7) that the RB4 antibody is able to strongly inhibit the migration of melanoma cells induced by ET-1. It may therefore represent a valuable starting point for the development of new tools for treatment of melanoma.

Moreover, the inventors herein showed (figure 5) that the RB4 antibody is efficiently internalized in endosomes and lysosomes together with ET_{B}. As ET_{B} is highly expressed in melanoma cells, the antibody of the invention could therefore be used for the specific delivery of cytotoxic molecules into melanoma cells, increasing antibody-mediated cell killing.

Thus, the RB4 antibody has the unique properties to i) inhibit the ET_{B} dependent G-protein signaling and migration of human melanoma cells, and ii) to be able to internalize cytotoxic or imaging compounds in human melanoma cells, therefore driving a high anti-tumoral efficacy and/or imaging potentiality. It is the first time that an antibody having such a high affinity for human ET_{B} is shown to display these two important properties.

These particular properties make the RB4 antibody a preferred tool for the development of immunological tools in the field of melanoma therapeutics.

The present invention therefore targets bi-functionalized Antibody-Conjugates (AC) comprising i) at least two different kinds of functional molecules (either two different imaging probes, or two different cytotoxic compounds, or one of each category) and ii) the variable domain of RB4 recognizing specifically the human endothelin receptor sub-type B (ET_{B}), that can specifically bind to the epitopes of SEQ ID NO:9 and SEQ ID NO:10 on human ET_{B} expressed on melanoma cells, that can inhibit in said cells G-protein signalling dependent from this receptor, that can be internalized in said cells in the endo-lysosomal pathway, yet without competing with its natural ligand ET1 and/or ET3. This AC can advantageously be used as a diagnostic and/or therapeutic anti-melanoma drug.

As used herein, the term "ET_{B}" designates the endothelin receptor of sub-type B, that is also abbreviated as ET-BR or ET-B, or ETRB, or HSCR2. It is encoded by the EDNRB gene (also called HSCR2 gene). The protein P24530 (SEQ ID NO:11) is a G protein-coupled receptor which activates a phosphatidylinositol-calcium second messenger system. Its ligand, endothelin, consists of a family of three potent vasoactive peptides: ET1, ET2, and ET3.

The term "antibody" is used herein in the broadest sense and specifically covers monoclonal antibodies (including full length monoclonal antibodies) of any isotype such as IgG, IgM, IgA, IgD, and IgE, polyclonal antibodies, multispecific antibodies, chimeric antibodies, and antigen-binding fragments. An antibody reactive with a specific antigen can be generated by recombinant methods such as selection of libraries of recombinant antibodies in phage or similar vectors, or by immunizing an animal with the antigen or an antigen-encoding nucleic acid.

A typical antibody is comprised of two identical light chains and two identical heavy chains that are joined by disulfide bonds. As used in the invention, the term "light chain" refers to mammalian immunoglobulin light chain, lambda (λ) or kappa (κ), having two successive domains: one constant domain and one variable domain. As used in the invention, the term "heavy chain" refers to chain of mammalian immunoglobulin denoted by: alpha (α), delta (δ), epsilon (ε), gamma (γ), and mu (µ). Each heavy chain has two regions, the constant region and the variable region. The constant region is identical in all antibodies of the same isotype. The variable region of each heavy chain is composed of a single Ig domain. The "variable region" or "variable domain" of an antibody refers to the amino-terminal domains of the heavy or light chain of the antibody. The variable domain of the heavy chain may be referred to as "V_{H}". The variable domain of the light chain may be referred to as "V_{L}". These domains are generally the most variable parts of an antibody and contain the antigen-binding sites. Each variable region contains three segments called "complementarity-determining regions" ("CDRs") or "hypervariable regions" ("HVRs"), which are primarily responsible for binding an epitope of an antigen and are interspersed with regions that are more conserved, designated "Framework Regions" (FR). The CDRs thus direct the specificity of the binding of the antibody. They are usually referred to as CDR1, CDR2, and CDR3, numbered sequentially from the N-terminus.

Each VH and VL is composed of three CDRs and four FRs, arranged from amino-terminus to carboxy-terminus in the following order: FR1, CDR1, FR2, CDR2, FR3, CDR3, FR4. The assignment of amino acid sequences to each domain is in accordance with well-known conventions (for example, the IMGT unique numbering convention as disclosed by Lefranc, M.-P.,et al., Dev. Comp. Immunol., 27, 55-77 (2003)). The functional ability of the antibody to bind a particular antigen depends on the variable regions of each light/heavy chain pair, and is largely determined by the CDRs. The variable region of the heavy chain differs in antibodies produced by different B cells, but is the same for all antibodies produced by a single B cell or B cell clone (or hybridoma).

By contrast, the constant regions of the antibodies mediate the binding of the immunoglobulin to host tissues or factors, including various cells of the immune system (e.g. effector cells) and the first component (Clq) of the classical complement system. Unlike the variable regions whose sequence varies greatly from one antibody to another, the constant regions are characterized by a very similar amino acid sequence from one antibody to another, which is characteristic of the species and isotype, with possibly some somatic mutations. The constant region of the heavy chain is composed of, from N-terminal to C-terminal, a CH1 domain, a hinge region, then CH2 and CH3 or CH2 to CH4 domains (depending on the isotype). The 'Fc fragment' naturally comprises the constant region of the heavy chain excluding the CH1 domain, that is, the lower hinge region and the constant CH2 and CH3 or CH2 to CH4 domains (depending on the isotype). The positions of the CH1 to CH3 or CH1 to CH4 domains and the hinge region of the different antibody isotypes from various species are known to those skilled in the art and can notably be found on the IMGT website. The constant region of the light chain (for antibodies possessing two heavy chains and two light chains) is composed of a single domain called CL.

The antibody RB4 identified by the inventors is a monoclonal IgG1 Kappa obtained from the hybridoma deposited at the CNCM (Collection Nationale de Cultures de Microorganismes, Institut Pasteur, 25 rue du Docteur Roux, F-75724 PARIS CEDEX 15) on 14 March 2023 under the accession number CNCM I-5937.

The AC of the invention preferably contains the light and heavy chains variable domains of this deposited monoclonal antibody.

As used herein, the term "monoclonal antibody" refers to an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody population arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one class and subclass, and light chains of only one type. Monoclonal antibodies are highly specific and are directed against a single antigen. An "antigen" is a predetermined molecule to which an antibody can selectively bind. The target antigen may be a polypeptide, a carbohydrate, a nucleic acid, a lipid, a hapten or any other naturally occurring or synthetic compound. Preferably, the target antigen is a polypeptide.

The CDR sequences of RB4 are listed in Table 1 and in the enclosed listing sequence:

**Table 1: amino acid sequences of SEQ ID NO:1-6**

| | SEQ ID NO : | Amino acid sequences |
|---|---|---|
| CDR1 V_{L} | 1 | QSLLDS DGKTY |
| CDR2 V_{L} | 2 | LVS |
| CDR3 V_{L} | 3 | WQGTHFPQT |
| CDR1 V_{H} | 4 | GYTFTDYT |
| CDR2 V_{H} | 5 | FYPGSDNI |
| CDR3 V_{H} | 6 | ARLVFFTGALDY |

The antibody RB4 comprises the light chain variable domain of SEQ ID NO:7 and the heavy chain variable domain of SEQ ID NO:8, as listed in Table 2 and in the enclosed listing sequence:

**Table 2: amino acid sequences of SEQ ID NO:7-8**

| | SEQ ID NO : | Amino acid sequences |
|---|---|---|
| Rendomab B4 light chain variable domain amino acid sequence | 7 | DIVMTQTPLT LSVIIGQPAS ISCKSSQSLL DSDGKTYLNW LLQRPGQAPK RLIYLVSKLD SGVPDRFTGS GSGTDFTLKI SRVEAEDLGV YYCWQGTHFP QTFGGGTKLE IK |
| Rendomab B4 heavy chain variable domain amino acid sequence | 8 | QVQLQQSGAE LVKPGASVKL SCKASGYTFT DYTIHWVKQR SGQGLEWIGW FYPGSDNIKY SERFKDKATL TADKSSSTVY MELSRLTSED SAVYFCARLV FFTGALDYWG QGTSVTVSS |

For complete information, the following polynucleotides can be used to encode the CDRs of the antibody contained in the AC of the invention:
- SEQ ID NO:12, corresponding to one polynucleotide encoding the CDR1 of the light chain of Rendomab-B4,
- SEQ ID NO:13 (ctggtgtct), corresponding to one polynucleotide encoding the CDR2 of the light chain of Rendomab-B4
- SEQ ID NO:14, corresponding to one polynucleotide encoding the CDR3 of the light chain of Rendomab-B4,
- SEQ ID NO:15, corresponding to one polynucleotide encoding the variable light chain of Rendomab-B4,
- SEQ ID NO:16, corresponding to one polynucleotide encoding the CDR1 of the heavy chain of Rendomab-B4,
- SEQ ID NO:17, corresponding to one polynucleotide encoding the CDR2 of the heavy chain of Rendomab-B4,
- SEQ ID NO:18, corresponding to one polynucleotide encoding the CDR3 of the heavy chain of Rendomab-B4,
- SEQ ID NO:19, corresponding to one polynucleotide encoding the variable heavy chain of Rendomab-B4.

Thus, as the AC of the invention contains the variable domain of RB4, the AC of the invention comprises :
a) a light chain comprising three CDRs of the sequences SEQ ID NO:1, 2 or 3, or having a sequence of at least 80%, preferably 85%, 90%, 95% and 98% identity with sequences SEQ ID NO:1, 2 or 3 after optimal alignment and
b) a heavy chain comprising three CDRs of the sequences SEQ ID NO: 4, 5 or 6, or having a sequence of at least 80%, preferably 85%, 90%, 95% and 98% identity with sequences SEQ ID NO: 4, 5 or 6 after optimal alignment.

Preferably, the AC of the invention contains an antibody variable domain recognizing specifically the human endothelin receptor sub-type B (ET_{B}), said variable domain comprising the CDRs of SEQ ID NO:1-6, or CDRs having a sequence of at least 90%, 95% and 98% identity with sequences SEQ ID NO:1-6 after optimal alignment.

In one embodiment, the AC of the invention comprises :
a) a light chain variable domain (V_{L}) of sequence SEQ ID NO: 7, or an amino acid sequence having at least 80%, preferably 85%, 90%, 95% and 98% identity with SEQ ID NO: 7 after optimal alignment and
b) a heavy chain variable domain (V_{H}) of sequence SEQ ID NO: 8, or an amino acid sequence having at least 80%, preferably 85%, 90%, 95% and 98% identity with SEQ ID NO:8 after optimal alignment.

By "optimal alignment with a preferred sequence", it is herein meant the two sequences have been aligned by means of a global alignment of the sequences in their entirety. This alignment is preferably performed by means of an algorithm that is well known by the skilled person, such as the one disclosed in Needleman and Wunsch (1970). Accordingly, sequence comparisons between two amino acid sequences or two nucleotide sequences can be performed for example by using any software known by the skilled person, such as the "needle" software using the "Gap open" parameter of 10, the "Gap extend" parameter of 0.5 and the "Blosum 62" matrix. Two sequences are "optimally aligned" when they are aligned so as to produce the maximum possible score for that pair of sequences, which might require the introduction of gaps in one or both of the sequences to achieve that maximum score. While optimal alignment and scoring can be accomplished manually, the process is facilitated by the use of a computer-implemented alignment algorithm, e.g., gapped BLAST 2.0, described in Altschul et al., (1997) Nucleic Acids Res. 25:3389-3402, and made available to the public at the National Center for Biotechnology Information website.

The invention also provides ACs containing antibodies or fragments whose amino acid sequences are or contains sequences that are "similar" or "substantially similar" to SEQ ID NO:1 to SEQ ID NO:8. "Similarity" of two targeted amino acid sequences can be determined by calculating a similarity score for the two amino acid sequences. As used herein, the "similarity score" refers to the score generated for the two sequences using the BLOSUM62 amino acid substitution matrix, a gap existence penalty of 11, and a gap extension penalty of 1, when the two sequences are optimally aligned. Two amino acid sequences are substantially similar if their similarity score exceeds a certain threshold value. The threshold value can be any integer ranging from at least 1190 to the highest possible score for a particular reference sequence (e.g., SEQ ID NO:1-8). For example, the threshold similarity score can be 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, or higher. If in a particular embodiment of the invention, the threshold score is set at, for example, 1300, and the reference sequence is any of SEQ ID NO:1-8, then any amino acid sequence that can be optimally aligned with any of SEQ ID NO:1-8 to generate a similarity score of greater than 1300 is be held as "similar" to SEQ ID NO:1-8. Amino acid substitution matrices and their use in quantifying the similarity between two sequences are well-known in the art and described, e.g., in Dayhoff et al. (1978), and in Henikoff et al. (1992). To generate accurate similarity scores using NCBI BLAST, it is important to turn off any filtering, e.g., low complexity filtering, and to disable the use of composition based statistics. One should also confirm that the correct substitution matrix and gap penalties are used.

An "epitope" is the site on the antigen to which an antibody specifically binds. It can be formed by contiguous residues or by non-contiguous residues brought into close proximity by the folding of an antigenic protein. Epitopes formed by contiguous amino acids are typically retained on exposure to denaturing solvents, whereas epitopes formed by non- contiguous amino acids are typically lost under said exposure.

RB4 has a high affinity for human ET_{B} expressed by CHO and for human ET_{B} naturally expressed by human melanoma cell lines (UACC-257, WM-266-4 and SLM8). More specifically, they have a dissociation constant (K_{D}) with human ET_{B} of SEQ ID NO:11 expressed on melanoma cells such as UACC-257 about 0,15 nM as determined by flow cytometry (Guava Easycyte Plus, Millipore). As used herein, the term "K_{D}" refers to the dissociation constant of a particular antibody/antigen interaction. As used herein the term "binding affinity" or "affinity of binding" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1: 1 interaction between members of a binding pair (e.g., antibody and antigen). The affinity of a molecule X for its partner Y can generally be represented by the dissociation constant (K_{D}). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

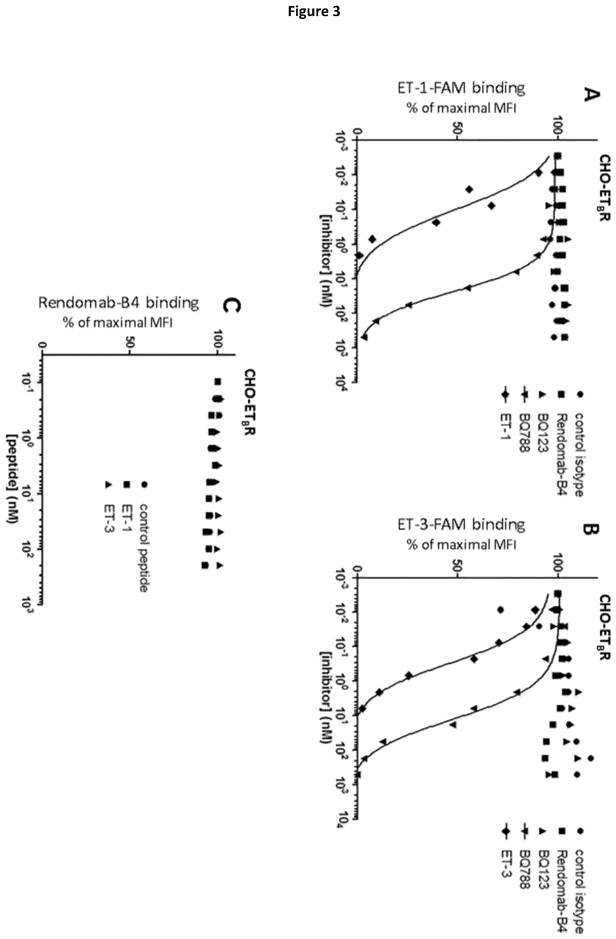

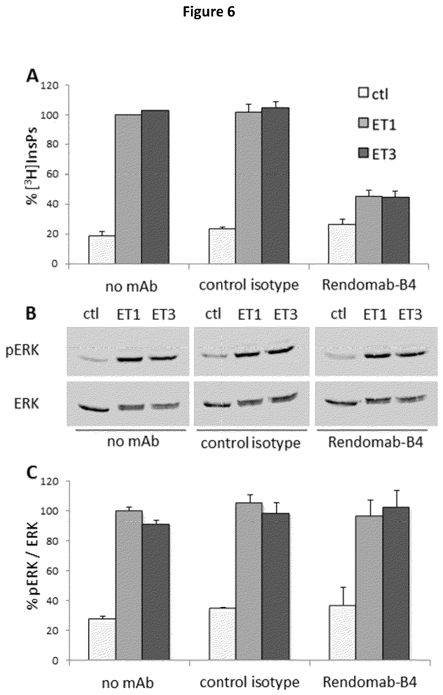

The present inventors have shown that the RB4 antibody binds preferentially on two sites of the N-terminal domain of ET_{B}. The first epitope has the amino acid sequence ERGFPPDRATP (SEQ ID NO:9) and corresponds to the most N-terminal region of the mature receptor, once the signal peptide is cleaved. The second epitope has the amino acid sequence EVPKGDRT (SEQ ID NO:10). The absence of any similarity between the two sequences suggests that RB4 recognizes a conformational epitope formed by the juxtaposition of two distinct regions of the receptor. These two sequences are unique to human ET_{B} and are not found in any other human proteins. Interestingly, the two sequences forming the epitope are not conserved between human and rodents, explaining why rendomab-B4 is unable to bind ET_{B}-expressing rat cells. Importantly, the RB4 antibody is capable of inhibiting the activation of ET_{B} by its natural ligands ET1 and ET3 without impairing their binding to the receptor (figures 3A, 3B and figure 3C). Specifically, it is capable of inhibiting the activating effect of ET1 and ET3 on protein G-dependent pathways (PLC activity and inositol phosphate production, figure 6A), but not the activating effect of ET1 and ET3 on ERK1/2 signaling (Figure 6B and C). In other words, it is capable of controlling differentially the various signalization pathways downstream of ET_{B}.

RB4 is the first reported mAb to possess the ability to differentially affect ET_{B}-coupled signaling pathways, behaving as a biased allosteric modulator inhibiting G protein-dependent processes but not β-arrestin-dependent ones.

The AC of the invention comprises a chimeric or a humanized antibody, or antigen-binding fragments thereof, which can be obtained by genetic engineering or by chemical synthesis, based on the RB4 murine antibody described above. More precisely, the AC of the invention comprises the same variable domain as the murine RB4 antibody, and therefore exhibits the same specificity and activity against ET_{B} as the RB4 antibody from which it derives.

The term "chimeric antibody" as used herein refers to an antibody containing a natural variable region (light chain and heavy chain) derived from an antibody of a given species in combination with constant regions of the light chain and of the heavy chain of an antibody of a species heterologous to said given species. Thus, a "chimeric antibody", as used herein, is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass. Such chimeric antibodies, or fragments of same, can be prepared by recombinant engineering. For example, the chimeric antibody could be produced by cloning recombinant DNA containing a promoter and a sequence coding for the variable region of a non-human monoclonal antibody of the invention, notably murine, and a sequence coding for the human antibody constant region. A chimeric antibody according to the invention coded by one such recombinant gene could be, for example, a mouse-human chimera, the specificity of this antibody being determined by the variable region derived from the murine DNA and its isotype determined by the constant region derived from human DNA.

In a particular embodiment, the AC of the invention contains a chimeric antibody comprising the variable domain of the murine RB4 antibody as disclosed above, and constant domains of human light and heavy chains of human immunoglobulins, e.g., the lambda or the kappa light chain of a human immunoglobulin, and the heavy chain of a human IgD, IgA, IgM, IgE or IgG.

For example, the AC of the invention can comprise :
- as light chain, the light chain variable domain (V_{L}) of sequence SEQ ID NO: 7 or an amino acid sequence having at least 80%, preferably 85%, 90%, 95% and 98% identity with SEQ ID NO: 7 after optimal alignment, and a human Ig Kappa or lambda constant domain,
- as heavy chain, the heavy chain variable domain (V_{H}) of sequence SEQ ID NO: 8 or an amino acid sequence having at least 80%, preferably 85%, 90%, 95% and 98% identity with SEQ ID NO: 8 after optimal alignment, and the CH1, CH2 and CH3 domains of a human IgG, IgM, IgE, IgD or IgA antibody.

In a more particular embodiment, the AC of the invention contains a chimeric antibody comprising the variable domain of the murine RB4 antibody as disclosed above, and the lambda light chain of a human immunoglobulin, and the CH1, CH2 and CH3 domains of a human IgG. In this case, the AC of the invention may comprise :
- as light chain, the light chain variable domain (V_{L}) of sequence SEQ ID NO: 7 or an amino acid sequence having at least 80%, preferably 85%, 90%, 95% and 98% identity with SEQ ID NO: 7 after optimal alignment, and a human Ig Kappa constant domain,
- as heavy chain, the heavy chain variable domain (V_{H}) of sequence SEQ ID NO: 8, or an amino acid sequence having at least 80%, preferably 85%, 90%, 95% and 98% identity with SEQ ID NO: 8 after optimal alignment, and the CH1, CH2, CH3 domains and hinge of the human IgG antibody.

In a preferred embodiment, the glycosylated residues present in the human immunoglobulin parts of the chimeric antibody are chemically removed or mutated, so as to improve its efficacy, and comply more easily with stability and regulatory issues. Glycosylated residues can be removed by enzymatic treatment with enzymes such as PNGase F (that cleave N-linked glycans from antibodies) or by chemical methods such as hydrazinolysis. These methods are well known in the art. Deglycosylation of the antibody is especially important when coupling of the functional molecules involve the use of an enzyme such as transglutaminase enzyme (see below).

In a preferred embodiment, the asparagine glycosylated residue(s) of the CH2 domain of the Fc region of human IgG have been mutated into non-glycosylated residues, more preferably into an Alanine (Ala or A), an Aspartic Acid (Asp or D) that do not affect the interaction of the IgG with the high-affinity FcγR, FcγRI/CD64.

In a more preferred embodiment, the asparagine glycosylated residues located in the CH2 domain of the Fc region of human IgG at position 297 (N297) of the heavy chains (amino acid numeration being done continuously in reference to the sequence displayed in SEQ ID NO:23 corresponding to the heavy chain of the antibody of the invention, comprising the heavy chain variable domain of RB4 and the CH1, CH2, CH3 and hinge domains of the human lgG1 antibody), have been mutated into Glutamine (Gln or Q), so as to allow an efficient coupling with a transglutaminase enzyme (see below).

In this case, the AC of the invention comprises :
- as light chain, the light chain variable domain (V_{L}) of sequence SEQ ID NO: 7 and a human Ig Kappa constant domain, eg., said light chain having e.g. the sequence SEQ ID NO:22,
- as heavy chain, the heavy chain variable domain (V_{H}) of sequence SEQ ID NO: 8 and the CH1, CH2, CH3 and hinge domains of the human IgG1 antibody, e.g., said heavy chain having e.g. the sequence SEQ ID NO:23.

In another aspect, the AC of the invention contains humanized variable domains containing the CDRs of RB4, or homologous sequences thereof, as disclosed in the prior application PCT/EP/ 2024/059388, which is herein incorporated by reference. In this AC, the CH2 domain of the Fc region of the human IgG contains naturally a Q297 (in a continuous numeration with respect to a sequence having the same variable domain size and IgG portions as SEQ ID NO:23).

In another specific embodiment, the AC of the invention contains fully human variable domains containing the CDRs of RB4 or homologous sequences thereof, as disclosed in the prior application PCT/EP/2024/059388, which is herein incorporated by reference. Preferably, in this AC, the CH2 domain of the Fc region of human IgG contains Q297 instead of N297 (in a continuous numeration with respect to a sequence having the same variable domain size and IgG portions as SEQ ID NO:23).

It is noteworthy that the antibody contained in the AC of the invention is coupled to the detection / therapeutic molecules by means of a trivalent linking platform which is coupled to the Q amino acid residue present in the antibody polypeptide chain, and not via a cysteine amino acid residue. It is therefore not the antibody conjugate disclosed in PCT/FR2024/050178.

Importantly, all these ACs covered by the invention specifically bind ET_{B} and inhibit its protein-G dependent subsequent signaling.

When the ACs contain human Ig constant parts, it is possible to introduce in the Fc constant part any mutation that is known to enhance the efficiency and reduce the adverse side effects of therapeutic antibodies. A number of these mutations are disclosed in PCT/EP/2024/059388. All of them are herein incorporated by reference.

### Bi-functionalized ACs based on RB4

An important characteristic of the antibodies of the invention is their ability to be internalized in endosomes and lysosomes of human tumoral cells such as melanoma cells (see Figure 5 and example below). They are therefore useful for targeting compounds into target tumor cells such as melanoma cells, in order to block their proliferation or kill them. They can also be used as imaging tools to label and/or quantify the tumor cells.

The objective of the present invention was the development of RB4-based bi-functionalized conjugates for the diagnosis and treatment of ET_{B}⁺ tumors. These bi-functionalized conjugates are capable of i) visualizing tumor cells through isotopic imaging (PET or SPECT) for diagnosis and patient selection for treatment; ii) destroying tumor cells using a cytotoxic molecule (e.g., MMAE) and a therapeutic radioactive isotope (e.g., Lu-177) and/or iii) monitoring treatment efficacy through isotopic imaging (PET or SPECT) following the principle of companion diagnostics.

Accordingly, the AC of the invention contains a trivalent linking platform which is able to bind covalently at least two different kinds of functional molecules, for example at least two cytotoxic and/or detection molecules. In one embodiment, each chain of the AC of the invention is covalently coupled through the platform to two cytotoxic molecules or to two detection molecules. In another embodiment, each chain of the AC of the invention is covalently coupled through the platform to one cytotoxic molecule and to one detection molecule.

In a first aspect, the present invention thus concerns an antibody conjugate (AC) comprising :
i) an antibody variable domain recognizing specifically the human endothelin receptor subtype B (ET_{B}),
ii) at least one trivalent linking platform able to bind two kinds of molecules chosen from cytotoxic and detection molecules, said platform being covalently coupled to the antibody part of the antibody conjugate.

The trivalent linking platform is preferably coupled to the two heavy chains of the antibody part of the AC of the invention. In this case, the AC of the invention contains two trivalent linking platforms (one on each heavy chain). The present invention also encompasses AC containing only one trivalent linking platform (when only one of the antibody chains is coupled to same). The present invention also encompasses AC containing more than two trivalent linking platforms, for example when the platforms are coupled to two Q amino acid residues present on each chain of the antibody. The AC of the invention can therefore contain one, two, three or four kind(s) of cytotoxic molecules (depending on the number of cytotoxic molecules coupled to the platform, and depending if one or two chains of the antibody are bound to the platform).

By "cytotoxic molecule", it is herein meant a toxin, a cytotoxic group or an effector group.

Said toxin molecule can be a ribosyl transferase, serine protease, guanyl cyclase activator, calmodulin- dependent adenyl cyclase, ribonuclease, DNA alkylating agent or mitosis inhibitor (e.g. doxorubicin) and the like, that may be used to target and kill melanoma cells. Said cytotoxic group can be a group directly or indirectly toxic for the cells targeted by the AC according to the present invention. By "directly cytotoxic", it is meant a group which is cytotoxic on its own. Direct cytotoxic group can be chosen in the group of the chemotherapeutic agents such as monomethyl auristatin E (MMAE), maytansinoid DM1, alkylating agents (mechlorethamine or chlorambucile; methotrexate; 5-fluoro-uracil; vinblastine; gemcitabine; fludarabine; nicotinamide; doxorubicin; mitomycin; L-asparaginase; cisplatin; duocarmycin, taxol, etc.). It can also be a cytotoxic polypeptide group such as ricin, abrin, Pseudomonas exotoxin, TNFα or interleukin 2. They are poorly cytotoxic as such but are able to give, in particular after an enzymatic reaction or an irradiation, a cytotoxic substance (or "drug"). It can be a DNA breaking agent such as caliceamicin, or a RNA polymerase II inhibitor such as alpha-amanitin. By "indirectly cytotoxic", it is meant a group which, although not cytotoxic on its own, can induce a cytotoxicity, for example by its action on another molecule or by a further action on itself. Indirectly cytotoxic agents ("prodrugs") can be cytotoxic chemotherapeutic agents such as methotrexate-alanine; mitomycin phosphate, 5-fluorocytosine, photofrin and capecitabine or cytotoxic polypeptide such as carboxypeptidase, aminopeptidase, endopeptidase, phosphatase, sulphatase, amidase, kinase, glycosidase, deaminase, reductase, or oxidase. The cytotoxic group can also be a nucleic acid molecule which is directly or indirectly cytotoxic such as an anti-sense oligonucleotide or an aptamer.

Said effector group can be a group capable of specifically recognizing a melanoma marker, or which makes it possible to recruit (i) an effector cell of the immune system i.e. NK cells, cytotoxic T cells, macrophages or (ii) the complement system. By "group capable of specifically recognizing a melanoma marker", it is meant, within the scope of the present invention, a ligand of a melanoma marker; an antibody (identical to or different from the antibody according to the present invention); a protein; a peptide; or a nucleic molecule such as a DNA, an RNA, an RNAi, an aptamer, a PNA or an LNA. By " melanoma label", both an ET_{B} and another membrane marker are contemplated. The effector group can recognize a melanoma marker such as S100, HMB-45 and Melan-A which is expressed at the surface of melanoma cells, thus ensuring better recognition specificity and thus increased targeting of melanoma cells. The effector group can alternatively exhibit a recognition specificity for a marker specifically present at the surface of effector cells of the immune system, i.e. NK cells, macrophages or cytotoxic T cells. Such a recruitment ensures targeted lysis of the melanoma cells recognized by the antibody of the present invention. The effector group can also have a recognition specificity for the complement system and, in particular, for protein C1 or its truncated form C1q, which initiates the cascade of molecular events which result in the death of the targeted melanoma cell. The effector group can also exhibit a recognition specificity for the complement system and, in particular, for protein C3 or its truncated form C3b, thus ensuring recruitment of effector cells of the immune system, which cells induce the death of the targeted melanoma cell. In a particular embodiment, it is possible to conjugate the antibody of the invention with a ERK1/2 inhibitor known in the art, such as TCS ERK 11e (Asundi J. et al, 2014) or with the BRAF inhibitors vemurafenib, dabrafenib and encorafenib that are used in the treatment of patients with BRAF-mutant melanoma (Proietti I. et al, 2020).

The AC of the invention can contain either one kind (in case the conjugate is intended for use in a single type of imaging) or two kinds (in case the conjugate is intended for use in multiple types of imaging detecting different physical phenomena with two different types of detectable or potentially detectable molecules, detection molecules or molecules that can become detectable. In a preferred embodiment, the AC of the invention contains two kinds of different detection molecules or molecules that can become detectable, particularly for diagnostic use of the conjugate.

By "detection molecule", it is herein meant a detectable label or a molecule that can become detectable (by further coupling). Said detection molecule is preferably an "easily detectable group", i.e., a group that can be detected by implementing an advantageously non-invasive appropriate detection technique such as microscopy, scintigraphy, positon emission tomography (PET), monophotonic emission tomography (SPECT), Cerenkov luminescence imagery (CLI), optic imaging, fluorescence imaging and magnetic resonance imaging (MRI). Each detection molecule is chosen based on the type of imaging for which the conjugate is intended.

Examples of detection molecules, which may be conjugated to the antibody of the AC of the invention, include:
a) a radioisotope (detectable molecule), a chelating agent (molecule that can become detectable, which becomes detectable after contact with a radiometal), or an affinity molecule (molecule that can become detectable after the AC comes into contact with a radioisotope conjugated to a binding partner of the affinity molecule, or with a chelating agent conjugated to a binding partner of the affinity molecule and then a radiometal). Such detection molecules are particularly useful when the conjugate is intended for use in isotopic imaging. The radioisotope can be conjugated covalently to the AC of the invention, or non-covalently by complexation with a chelating agent covalently conjugated to the AC of the invention. Radioisotopes or radiometals that emit gamma rays directly (direct emission of a gamma photon during decay) or indirectly (emission of a positron during decay, which after annihilation with a surrounding electron leads to the emission of 2 gamma rays) are preferred because they are detectable by positron emission tomography (PET) or single-photon emission computed tomography (SPECT). The label can be a radioactive isotope such as actinium-225, iodine-123, iodine-125, iodine-126, iodine-133, indium- 111, indium-113m, bromine-77, gallium-67, gallium-68, lutetium-177, ruthenium-95, ruthenium-97, technetium-99m, fluorine-18, radium-223, scandium- 47, tellurium-122m, thulium-165, yttrium-90 and yttrium-199. It should be observed that some of these radioactive atoms can also be cytotoxic groups because of the radioactivity quantity they can deliver.

Examples of radiometals that emit gamma rays directly or indirectly (via the emission of a positron, which after annihilation with a surrounding electron leads to the emission of 2 gamma rays) include ^{99m}Tc, ¹¹¹In, ⁶⁴Cu, ⁶⁸Ga, ⁸⁹Zr, and ⁴⁴Sc. ^{99m}Tc and ¹¹¹In are direct emitters of gamma rays detectable by single-photon emission computed tomography (SPECT), while ⁶⁴Cu, ⁶⁸Ga, ⁸⁹Zr, and ⁴⁴Sc are indirect emitters of gamma rays detectable by positron emission tomography (PET). Another radioisotope that can be used in PET imaging is ¹⁸F, which is a positron emitter (and therefore an indirect emitter of gamma photons) and can be introduced onto prosthetic groups, known to those skilled in the art, which can be conjugated to the AC of the invention.

In a preferred embodiment, the radiometals used in the AC of the invention are of therapeutic value or for imagery purposes. Therapeutic radiometals that can be used in the AC of the invention are for example: ²²⁵Ac, ²¹²Pb, ²¹¹At, ²¹²Bi, ²¹³Bi, ¹⁵²Tb, ¹⁶⁰Tb, ¹⁶¹Tb, ²²⁷Th, ²²³Ra, ²²⁴Ra, ¹⁷⁷Lu, ⁹⁰Y, ⁶⁷Cu, ⁴⁷Sc, ¹⁵³Sm, ¹⁶⁶Ho, ¹⁸⁶Re, ¹⁸⁸Re, ⁸⁹Sr, ^{117m}Sn, ¹¹¹In, ¹⁹⁸Au, and ⁵⁸Co. Imagery or detection radiometals that can be used in the AC of the invention are for example : ⁶⁷Ga, ⁶⁸Ga, ¹¹¹ In, ⁴⁴Sc, ⁶⁰Cu, ⁶¹Cu, ⁶²Cu, ⁶⁴Cu, ⁸⁴Zr, ⁸⁶Zr, ⁸⁷Zr, ⁸⁹Zr, ⁶³Zn, ⁴³Sc, ⁴⁴Sc, ¹⁹²Au, ¹⁹³Au, ¹⁹⁴Au, ¹⁹⁶Au, ⁵¹Mn, ⁵²Mn, ^{52m}Mn, ¹⁴⁸Tb, ¹⁵¹Tb, ^{151m}Tb, ¹⁵²Tb, ⁴⁵Ti, ⁶⁵Ga, ⁶⁶Ga, ⁶⁷Ga, ^{99m}Tc, ⁵⁵Co, ⁸⁰Sr, ⁸¹Sr, ⁸³Sr, ⁷⁰As, ⁷¹As, ⁷²As, ⁷⁴As, ⁸¹Rb, ^{82m}Rb, ⁵²Fe, ⁸⁶Y, [¹⁸F]AlF.

The radiometals are typically conjugated indirectly to the AC of the invention by means of a chelating agent that binds the radiometal. Examples of chelating agents include linear or cyclic chelating agents, such as 1,4,7-triazacyclononane-1,4,7-triacetic acid (NOTA), dodecane tetracetic acid (DOTA), 1,4,7, 10-tetrakis(carbamoylmethyl)- 1 ,4, 7, 10-tetracyclododecane (TCMC), 1-N-(4-aminobenzyl)-3,6,10,13,16,19-hexazabicyclo[6.6.6]eicosane-1,8-diamine (DiAmSar), N,N-bis(2-hydroxybenzyl)ethylenediamine-N,N-diacetic acid (HBED), deferoxamine (DFO), and diethylenetraminepentacetic acid (DTPA), N,N'-bis[(6-carboxy-2-pyridil)methyl]- 4,13-diaza-18crown-6 (MACROPA), 2,2' ,2"-nitrilotriacetic acid (NTA), 2,2- bis(hydroxymethyl)-2,2',2"-nitrilotriethanol (BisTris), ethylene glycol-bis(2-aminoethyl ether)-N,N,N',N'-tetraacetic acid (EGTA), ethylenediamine-N,N,N',N'-tetraacetic acid (EDTA), 1,2-bis(2-aminophenoxy)ethane-N,N,N',N'-tetraacetic acid (BAPTA), 1,4,7,10- tetraazacyclododecane-1,7-diacetic acid (DO2A), 1,4,7,10-tetraazacyclododecane-1, 4,7- triacetic acid (DO3A), desferrioxamine B, and their derivatives. Preferred derivatives of DOTA and NOTA include DOTAGA and (R)-NODAGA, respectively. The use of a chelating agent is advantageous as it allows for the storage of the ready-to-chelate conjugate, to which the radioisotope, which may have a very short half-life, can be added only at the last moment before use. In this case, complexation can be performed just before using the conjugate of the invention for an isotopic imaging application. Although the conjugation to the chelating agent can be indirect via an affinity molecule and its binding partner, the AC of the invention contains advantageously a directly conjugated chelating agent.

b) a fluorophore or an affinity molecule that can become detectable (when the AC comes into contact with a fluorophore conjugated to a binding partner of the affinity molecule).

Fluorophores are useful when the AC is intended for use in fluorescence imaging. Similar to radiometals in the case of radioisotopes, the fluorophore can be covalently conjugated to the AC, or non-covalently when the AC is conjugated to an affinity molecule whose binding partner complexes with the fluorophore.

Examples of fluorophores that can be detected in fluorescence imaging and therefore conjugated to the mutant antibody fragment according to the invention include compounds from the cyanine family (including Cyanine3, Cyanine5, Cyanine5.5, Cyanine7, Cyanine7.5, with or without water-solubilizing groups such as sulfonates); compounds from the xanthene family (including compounds from the rhodamine family such as X-rhodamine, rhodamine B, and compounds from the fluorescein family, with or without water-solubilizing groups such as sulfonates); compounds from the coumarin family (including hydroxycoumarin, aminocoumarin, methoxycoumarin, with or without water-solubilizing groups such as sulfonates); and compounds from the boron-dipyrromethene (BODIPY) family, including aza-BODIPY derivatives. Preferably, the fluorophore is selected from Cyanine5, Cyanine7, a rhodamine, or an azaBODIPY. The label can also be a fluorescent, chimiofluorescent or bioluminescent label such as fluorescein and derivatives thereof, rhodamine and derivatives thereof, GFP (Green Fluorescent Protein) and derivatives thereof, umbelliferone; luminol; luciferase or luciferin.

c) a chromophore (detectable molecule) or an affinity molecule (molecule that can become detectable after the AC conjugated to the affinity molecule comes into contact with a chromophore conjugated to its binding partner).

Chromophores are useful when the conjugate is intended for use in optical imaging by absorbance measurement. The chromophore can be covalently conjugated to the AC of the invention, or non-covalently when it is already conjugated to a binding partner of the affinity molecule that is conjugated covalently to the AC. Examples of chromophores that can be conjugated to the AC of the invention for use in optical imaging include phenolphthalein, gentian violet, or Congo Red.

Thus, the detection molecules contained in the AC of the invention are preferably selected from: a fluorophore, a chromophore, an affinity molecule, or a radioisotope (this latter being preferably coupled to the platform of the invention by means of a chelating agent).

It is preferred that the functional molecules are linked to the antibody via a covalent bond, directly or indirectly. However, in certain embodiments, they may be linked to the antibody via a strong non-covalent bond, for example by using affinity molecules.

The affinity molecule can become detectable by indirect means. It can be an enzyme or a molecule capable of generating a detectable and possibly quantifiable signal under particular conditions (such as when putting into contact with an adapted substrate). By way of illustrating and non-limiting examples, this affinity molecule can be chosen in the group consisting of: biotin, streptavidin, digoxigenin, 5-bromodeoxiuridin, alkaline phosphatase, peroxidase, acetylcholine esterase (AChE), glucose amylase, lysozyme, and their ligand. Examples of affinity molecules include biotin (capable of forming a complex with streptavidin conjugated to a fluorophore, chromophore, radioisotope, or chelating agent), avidin (capable of forming a complex with biotin conjugated to a fluorophore, chromophore, radioisotope, or chelating agent), and streptavidin (capable of forming a complex with biotin conjugated to a fluorophore, chromophore, radioisotope, or chelating agent).

These detection molecules enable in particular sites at which the ET_{B} is over-expressed to be identified and localized because of the ET_{B} binding specificity of the AC of the invention. Irrespective of the technique used to conjugate the antibody with a cytotoxic group, the only requirement to meet within the scope of this conjugation is thus that the conjugated antibody preserves its ET_{B} binding specificity and its antagonist property.

In a preferred embodiment, the AC of the invention contains two kinds of detection molecules as defined above, for example:
1) a radioisotope for use in isotopic imaging and a fluorophore or an affinity molecule for use in fluorescence imaging; or
2) two detection molecules or molecules that can become detectable of the same type (and preferably identical), to increase the detected signal.

In another preferred embodiment, the AC of the invention contains one detection molecule and one cytotoxic agent as defined above, for example ⁸⁹Zr and ¹⁷⁷Lu. Lu-177 emits beta particles and gamma rays, which can damage and kill cancer cells when delivered directly to the tumor site. This is typically achieved by conjugating Lu-177 to a targeting molecule, such as an antibody or peptide, which specifically binds to tumor cells. Once bound, the radioactive decay of Lu-177 emits radiation that destroys the cancer cells locally. Zirconium-89 (Zr-89) is a radioactive isotope that is primarily used in positron emission tomography (PET) imaging rather than as a cytotoxic agent. It is chosen for its favorable physical properties for imaging, such as a relatively long half-life of approximately 78.4 hours, which allows for extended imaging times and is particularly useful for tracking biological processes over several days. In this embodiment, the AC of the invention, after conjugation and radiolabeling, will carry one therapeutic cytotoxic molecule (DOTAGA / Lu-177) and one imaging molecule (DFO/Zr-89). Their applications include: stratification of patients eligible for treatment and monitoring therapeutic efficacy through isotopic imaging (see below), as well as using an original combination therapy (cytotoxic molecule / therapeutic radioisotope) to treat patients.

In another preferred embodiment, the AC of the invention contains two kinds of cytotoxic molecules as defined above, for example MMAE and a DOTAGA/Lu-177 complex, as described in the examples below.

In specific embodiments, the drug and/or imaging probe is / are conjugated to the antibody of the invention through a coupling chemical agent. These coupling chemical agents are preferably cyclooctyne derivatives, such as DBCO (dibenzylcyclooctyne), DIBAC (diethylaminocyclooctine, or BARAC), TCO (trans-cyclooctene), BCN (bicyclo[6.1.0]nonyne), that can be coupled to azide or tetrazine groups by strain-promoted azide-alkyne cycloaddition or by IEDDA (click chemistry). It is also possible to perform the coupling reaction *in vivo*, using a pre-targeting strategy.

All the cytotoxic and detection molecules mentioned above are hereafter referred to as "functional molecules of interest". By extension, the intermediary chelating or coupling chemical agents described above, are also designated as "functional molecules of interest" that they will allow to attach the former onto the antibody of the AC.

In the AC of the invention, each chain of the antibody part can be covalently coupled to two functional molecules of interest. In particular, each chain of the antibody part can be coupled to one or two cytotoxic molecules, one or two detection molecules or to one cytotoxic molecule and one detection molecule.

It is possible that the functional molecule of interest, e.g., a cytotoxic molecule, is coupled to the trivalent linking platform without the means of a coupling agent. In this case, the molecule of interest, e.g., the cytotoxic drug, is directly coupled to the trivalent linking platform.

It is also possible to couple the antibody part of the AC of the invention to a platform which is bound to one chelating agent and/or a coupling agent. In this case, the AC can be injected *in vivo* as pre-targeting therapies and may be used as target to bind drugs administered afterwards. The drug, when and once injected later on, will bind covalently the antibody by means of the coupling agent present in the platform (e.g., via a CLICK reaction).

In a particular aspect, the invention refers to a kit containing:
i) an antibody coupled to one coupling function or two coupling functions by means of the platform,
ii) one or two appropriate functional molecule(s) that can be attached to the antibody via click chemistry depending on the intended use (therapeutic and/or detection)
and the separate, staggered, or simultaneous therapeutic/diagnostic use of these two products for the treatment/detection of melanoma and other solid cancers in which the human endothelin B receptor is overexpressed, such as glioblastoma, pancreatic cancer, ovarian cancer, bladder cancer, lung cancer, kidney cancer, or vulvar cancer or liquid cancers in which the human endothelin B receptor is overexpressed as leukemia, lymphoma, and myeloma.

The AC of the invention may additionally be conjugated to a pharmacomodulatory molecule. Examples of pharmacomodulatory molecules include linear or branched chains of poly(ethylene glycol), linear or branched chains of poly(glutamic acid), cholesterol, and molecules capable of binding to albumin (e.g., fatty acids, metal ions, bilirubin, a toxin, p-iodophenyl butyryl, biphenyl group). Such conjugation can improve the pharmacokinetics of the AC of the invention.

The AC of the invention may also be conjugated to other functional molecules with alternative means of the prior art.

### Conjugation processes

Those skilled in the art know different techniques enabling such molecules to be conjugated with an antibody. These techniques allow a covalent coupling between the antibody and the functional molecules of interest by taking advantage of particular chemical groups carried by the antibody and by the functional molecules of interest. Among these particular chemical groups, a thiol group, an ester group, an amino group, an acid group and any chemical element likely to be implemented in "click-chemistry" can be mentioned. These techniques have been extensively described in the prior art, for example by Costoplus et al. (Peptide-Cleavable Self-immolative Maytansinoid Antibody-Drug Conjugates Designed To Provide Improved Bystander Killing. ACS Med Chem Lett. 2019 Sep 27;10(10):1393-1399), Sonzini et al. (Improved Physical Stability of an Antibody-Drug Conjugate Using Host-Guest Chemistry. Bioconjug Chem. 2020 Jan 15;31(1):123-129), and Dickgiesser et al. (Site-Specific Conjugation of Native Antibodies Using Engineered Microbial Transglutaminases. Bioconjug Chem. 2020 Mar 12. doi: 10.1021/acs.bioconjchem.0c00061).
**Random Conjugation** refers to methods where functional molecules are attached to the antibody at multiple and undefined sites. The conjugation does not distinguish between different sites on the antibody, leading to a non-uniform product. Random conjugation may involve lysine-based conjugation, using NHS esters, squaramate or isothiocyanate (targeting the primary amine groups, present on lysine residues and the N-terminus of the antibody).

**NHS ester Conjugation** typically employs NHS-esters (N-Hydroxysuccinimide esters), which react with amine groups to form stable amide bonds. It is a straightforward and efficient process and lysine residues are abundantly present on the surface of antibodies. Due to the abundance of lysine residues, the conjugation can lead to unwanted modifications in critical regions of the antibody, potentially affecting its binding affinity and activity.

**Squaramate groups** are derivatives of squaric acid, characterized by a four-membered ring structure containing two carbonyl groups and two hydroxyl groups. When modified into squaramate or squarate esters, these molecules can react with nucleophiles such as amines and thiols, forming stable amide or ester bonds. Squaramate groups can conjugate with both amino (-NH2) and thiol (-SH) groups, offering versatility in the choice of conjugation targets on the antibody. This dual reactivity expands the scope of potential modifications and applications. Similar to NHS-esters and isothiocyanates, squaramate derivatives can be used for random conjugation by targeting the lysine residues on antibodies. The reaction typically involves the formation of stable amide bonds with the amine groups on lysines. Yet, the reaction is non-specific, and the resulting conjugates may have variable binding affinities and functionalities.

**Isothiocyanate Conjugation** involves functional molecules containing isothiocyanate groups that react with amine groups to form stable thiourea linkages. This method is relatively simple and can be used to attach a wide variety of functional groups to antibodies. Yet, the reaction is non-specific, and the resulting conjugates may have variable binding affinities and functionalities.
To overcome these hurdles, **targeted conjugation methods** have been designed to attach functional molecules to specific sites on the antibody. This leads to more uniform products and often preserves the antibody's ability to bind its antigen. Moreover, site-specific conjugation techniques allow: i) perfect control over the ratio of different molecules of interest coupled to the antibody, ii) control over the number and location of conjugates on the protein and thus the homogeneity of the conjugates (vs. conjugates obtained by random conjugation methods), iii) ensuring robust reproducibility of batches of biopharmaceuticals produced by this approach. Targeted conjugation methods include site-specific cysteine modification, enzymatic conjugation.

**Site-specific cysteine modification** targets reduced cysteine residues that are generated by the reduction of disulfide bonds in the antibody. The functional molecules to be coupled typically contain maleimide or iodoacetamide or groups that react with thiol groups (from cysteine) to form stable thioether bonds. This technic provides better control over the conjugation process, resulting in homogeneous products and preserving the antibody's binding affinity. As additional steps are required to reduce the disulfide bonds, care must be taken to ensure that the antibody's structural integrity is maintained.

**Enzymatic conjugation methods** utilize specific recognition sites that are naturally present in the antibody polypeptide sequence, or that have been introduced into the antibody by genetic engineering. Enzymes such as glycotransferases, transglutaminase or sortase are used, which recognize unique peptide sequences and facilitate the conjugation of functional molecules to these specific sites. Highly specific and precise conjugation is achieved, leading to uniform products with preserved antibody functionality. Among the available enzymes, microbial transglutaminase (mTGase) from the species *Streptomyces mobaraensis* has found increasing interest as an attractive alternative to conventional chemical protein conjugation of functional moieties including antibodies. The MTG catalyzes under physiological conditions a transamidation reaction between a 'reactive' glutamine of a protein or peptide and a 'reactive' lysine residue of a protein or peptide, whereas the latter can also be a simple, low molecular weight primary amine such as a 5- aminopentyl group (Jeger S. et al., 2010, Angew. Chem. Int. Ed., 49, 9995-9997). Advantageously, the CH2 domain of the Fc region of human IgG contains at least one glutamine residue (Q) that can be used by the transglutaminase enzyme.

Any of these methods can be used to bind the functional molecules of interest to the antibody part of the AC.

The AC of the invention contains an antibody part which is conjugated to at least two kinds of molecules of interest. These two molecules of interest are typically conjugated to the same amino acid residue of the antibody, by means of a trivalent linking chemical entity that is used as an adaptor to couple the two molecules onto the same amino acid residue of the antibody. This latter option, which is herein preferred, enable to ensure the same biodistribution, and avoids heterogenous biodistribution of the functional molecules of interest. This chemical entity can be any trivalent linking moiety described in the art, in particular any trivalent moiety molecules proposed in WO2022/266499 (MAYO FOUNDATION FOR MEDICAL EDUCATION AND RESEARCH), or any biocompatible modular tetrazine molecules described in US2020/354381 (CNRS and UNIVERSITE DE BOURGOGNE), these two documents being incorporated herein by reference.

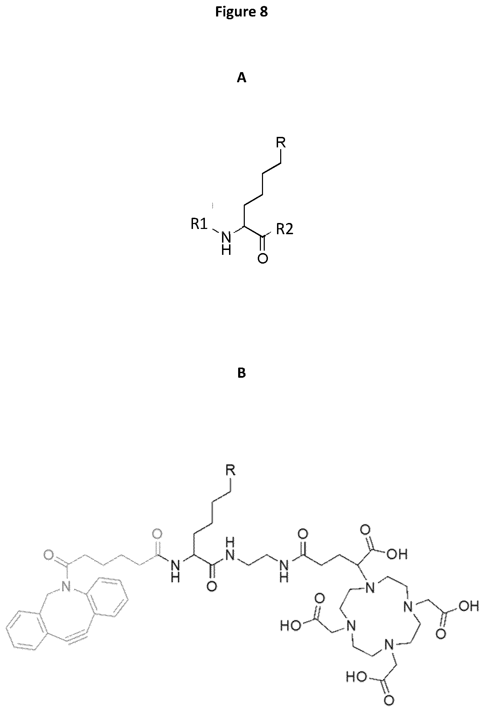

In a preferred embodiment, the trivalent linking moiety used to attach the at least two functional molecules of interest to the antibody of the AC of the invention is a lysine moiety which has been functionalized with two functional molecules or intermediary chelating or chemical agents as described above (see figure 8). This lysine residue can be used together with a microbial transglutaminase enzyme (mTGase), to attach the functional molecules of interest to the glutamine residue located in the antibody part of the AC of the invention, in particular in the heavy chain(s) of the chimeric RB4-based antibody disclosed above (more precisely, in the CH2 domain of the constant part Fc of the fuman IgG antibody). This conjugation process is well known in the art, see e.g. WO2023/072934 (ARARIS BIOTECH), which is incorporated by reference. The lysine moiety can also contain a chemical coupling agent such as a cyclooctyne derivative as mentioned above, that can react with azide-containing residues of the antibody molecule which have been obtained by removing the glycan molecules (Adumeau et al., 2018, Adumeau et al., 2022).

Figure 8A shows the structure of a preferred trivalent lysine moiety that can be used to attach two molecules of interest (or chemical / chelating agents intended to bind to these molecules) to the antibody part of the AC of the invention.
- If R is NH₂, then the microbial transglutaminase mTGase can be used to covalently bind the lysine residue on the Q amino acid of the CH2 domain of the constant part Fc of the human IgG antibody. In this case, it may be advantageous to remove the glycosylated residues to enhance the efficiency of the enzymatic coupling process. As Jeger et al. showed that glycosylated antibodies could not be conjugated efficiently, it is also recommended to mutate the other asparagine glycosylated residue(s) present in the heavy chain into Q residue(s) so as to promote the coupling of two couples of molecules (Jeger S. et al., 2010, Angew. Chem. Int. Ed., 49, 9995-9997).
- If R is maleimide, conjugation can be performed on the thiol groups of the cysteine -residues of the antibody.
- If R is an isothiocyanate or squaramate group, the molecules of interest (or chemical / chelating agents intended to bind to these molecules) can be conjugated to the lysine residues of the antibody.
- If R is a cyclooctyne derivative as disclosed above (DBCO, BCN, etc.), conjugation can be performed on azide groups that are produced on the antibody chains after glycans have been removed (Adumeau et al., 2018, Adumeau et al., 2022).

In the trivalent moiety disclosed in Figure 8A, R1 and R2 can be any chelating agent, coupling agent or functional molecules of interest disclosed above.

To conclude, the AC of the invention preferably contains two functional molecules of interest that are covalently bound to the amino acid Q residue of the antibody polypeptide by means of a trivalent linking platform interacting with the Q residue. In a preferred embodiment, the functional molecules of interest are bound to the platform directly, i.e., without requiring any intermediary chemical coupling agent. In an alternative embodiment, the trivalent linking platform is not directly conjugated to the two functional molecules of interest, but is conjugated to one or two chemical coupling agents, that are able to complex or covalently link (click chemistry) the functional molecules of interest, as described above. This trivalent linking platform is even more preferably a lysine trivalent moiety as shown on figure 8.

In a particular example of the AC of the invention, the two functional molecules of interest are conjugated to the antibody part by means of a trivalent linking moiety (e.g. a lysine moiety) containing DOTAGA or DFO as chelating agent and/or DBCO as chemical coupling agent. The radioactive compound Lu-177 can thereafter be complexed by the chelating agent DOTAGA, the Zr-89 radioisotope can be complexed by the chelating agent DFO and the cytotoxic drug MMAE can be coupled to the DBCO compound.

Preferred AC bi-functionalized conjugates of the invention thus include:
- ACs containing a chimeric RB4 antibody which is covalently conjugated to DOTAGA/Lu-177 and DBCO/MMAE on position Q297 by means of a lysine moiety (see figure 8B),
- ACs containing a chimeric RB4 antibody which is covalently conjugated to DBCO/MMAE and DFO/Zr-89 on position Q297 by means of a lysine moiety.

Other preferred ACs of the invention include:
- ACs containing a chimeric RB4 antibody which is covalently conjugated to DOTAGA and MMAE by means of a lysine moiety which is coupled to a Q amino acid residue of the antibody part,
- ACs containing a chimeric RB4 antibody which is covalently conjugated to MMAE and DFO by means of a lysine moiety which is coupled to a Q amino acid residue of the antibody part.

As the two Fc chains of the AC of the invention contains a Q residue, the transglutaminase enzyme may covalently attach two functional molecules of the invention on each Q residue. This means that the AC of the invention may carry four cytotoxic agents (two on each Q residue), or four detection agents (two on each Q residue), or two cytotoxic agents and two detection agents (one couple of cytotoxic & detection molecules on each Q residue).

In the preferred embodiment where an asparagine glycosylated residue has been mutated into a Q residue, then each polypeptide chain of the antibody part of the AC of the invention contains at least two Q residues, so that two couples of functional molecules may be conjugated on each chain of the antibody (the transglutaminase enzyme will add the lysine residue coupled to two functional molecules on the two glutamine residues present in each Fc chain of the IgG antibody). In this case, the AC of the invention may potentially carry eight cytotoxic agents (four on each Q residue), or eight detection agents (four on each Q residues), or four cytotoxic agents and four detection agents (one couple of cytotoxic & detection molecules on each Q residue, one couple of cytotoxic & detection molecules on each Q residue), or any other combination thereof.

Thus, the AC of the invention can carry at least two different kinds of functional molecules as defined above, but the actual number of functional molecules per antibody can vary depending on the conjugation process that has been chosen and on the structure of the chimeric antibody.

### ACs with fragments

An "antibody fragment" comprises a portion of an intact antibody, preferably the antigen binding and/or the variable region of the intact antibody. Examples of antibody fragments include Fab, Fab', F(ab')₂ and Fv fragments; diabodies; linear antibodies (see U.S. Pat. No. 5,641,870); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (CH1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having different antigen-binding activity and is still capable of cross-linking antigen. Fab' fragments differ from Fab fragments by having a few additional residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known. The term "Fv" as used herein refers to the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three hyper variable regions specific for an antigen) has the ability to recognize and bind antigen, although at a lower affinity than the entire binding site. "Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding.

In a preferred embodiment, the AC of the invention contains a functional fragment of the RB4 antibody as disclosed above, or of the chimerized or humanized version thereof, as disclosed above. This "functional fragment" preferably comprises the antigen binding or variable region of the RB4 intact antibody and retains the same binding specificity as the RB4 antibody from which it arises and sufficient affinity, preferably at least equal to 1/100, more preferably at least 1/10 of RB4.

More particularly, the AC of the invention contains a functional fragment such as functional Fv, Fab, (Fab')₂, Fab', scFv, scFv-Fc and diabodies, or any fragment of the RB4 antibody as disclosed above, whose half-life has been preferably increased by chemical modification. This chemical modification can be the addition of polyalkylene glycol such as polyethylene glycol (PEGylation) (PEGylated fragments are referred to as Fv-PEG, scFv-PEG, Fab-PEG, F(ab')₂-PEG and Fab'-PEG), and also include their incorporation in a liposome, microsphere or Poly D, L-lactic-co-glycolic acid (PLGA). These fragments will possess at least the six CDRs of RB4 (or functional homologs thereof) so that they are capable of exerting the same activity, even partial, as RB4.

These functional fragments can be obtained from the RB4 or chimerized or humanized or human antibodies derived thereof by methods such as enzyme digestion, including pepsin or papain, and/or by cleavage of the disulfide bridges by chemical reduction. They can be also obtained by recombinant genetics techniques also known to a person skilled in the art or by peptide synthesis by means, for example, of automatic peptide synthesizers such as those sold by Applied BioSystems, etc.

In a preferred embodiment, the present invention relates to an AC containing a Fab fragment of the chimeric antibody disclosed above, namely a Fab fragment comprising:
- as light chain, the light chain variable domain (V_{L}) of sequence SEQ ID NO: 7 and a human Ig Kappa or lambda constant domain,
- as heavy chain, the heavy chain variable domain (V_{H}) of sequence SEQ ID NO: 8 and the CH1 domain of a human IgG, IgA, IgM, IgD or IgE antibody.

It is noteworthy that the antibody functional fragment contained in the AC of the invention is not the Fab fragment disclosed in PCT/FR2024/050178, as the trivalent platform is coupled to a Q residue and not to a cysteine residue.

In an even more particular embodiment, the present invention relates to an AC containing a Fab fragment of the chimeric antibody disclosed above, said fragment comprising :
- as light chain, the light chain variable domain (V_{L}) of sequence SEQ ID NO: 7 and a human Ig Kappa constant domain, said light chain having e.g. the sequence SEQ ID NO:24,
- as heavy chain, the heavy chain variable domain (V_{H}) of sequence SEQ ID NO: 8, the CH1 domain of the human IgG1 antibody and the human IgG1 hinge, said heavy chain having e.g. the sequence SEQ ID NO:25.

In a very particular embodiment, when the conjugation of the cytotoxic or imaging agents involves the use of a transglutaminase enzyme (mTGase) as described above, it is recommended to link a "Q tag" (i.e., a small peptide containing a glutamine residue Q) to antibodies fragments, so that a lysine-containing trivalent platform coupled to the at least two cytotoxic and/or detection molecules can covalently bind the antibody fragment of the invention (Fab, scFv, diabody, minibody, or nanobody) by interacting with said glutamine residue. This is described in the study of Strop P, Bioconjugate Chem. 2014, 25, 855-862.

Said Q tag is preferably covalently coupled to the light chain of this antibody fragment. In this case, the present invention would relate to an AC containing a Fab fragment comprising:
- as light chain, the light chain variable domain (V_{L}) of sequence SEQ ID NO: 7, the human Ig Kappa constant domain and a Q tag having the sequence LLQGA (SEQ ID NO:27), said light chain having e.g. the sequence SEQ ID NO:26,
- as heavy chain, the heavy chain variable domain (V_{H}) of sequence SEQ ID NO: 8, the CH1 domain of the human IgG1 antibody, and the human IgG1 hinge, said heavy chain having e.g. the sequence SEQ ID NO:25.

The Q tag can also be coupled to the heavy chain of the antibody fragment.

### Uses of the AC of the invention

### Therapeutic uses

An important characteristic of the ACs of the invention is their ability to target and be internalized by melanoma cells. Depending on the nature of functional molecules they carry (cytotoxic and/or imaging molecules), they can be used in the treatment of a subject and/or in diagnosing a disease or condition in a subject.

If the AC carries at least one cytotoxic molecule, the AC of the invention can be used in a pharmaceutical composition. If the AC according to the invention is used in the treatment of cancer, it is preferred that it comprises at least one cytotoxic molecule that has the potential to kill or inhibit the proliferation of the tumor cell to which the AC binds to. In certain embodiments, this cytotoxic molecule (e.g., a toxin or MMAE) exhibits its cytotoxic activity after the AC has been internalized into the tumor cell.

In another aspect, the present invention therefore relates to a pharmaceutical composition comprising the AC of the invention, as described above, and a pharmaceutically-acceptable carrier. Preferably, the pharmaceutical composition of the invention contains, in addition to the AC of the invention, various diluents, fillers, salts, buffers, stabilizers, solubilizers, and other materials well known in the art.

As used herein, "pharmaceutically acceptable carrier" includes any and all solvents, buffers, salt solutions, dispersion media, coatings, antibacterial and antifungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion. The use of media and agents for pharmaceutically active substances is well known in the art. A typical pharmaceutical composition for intravenous infusion could be made up to contain 250 ml of sterile Ringer's solution, and 100 mg of the combination. Actual methods for preparing parenterally administrable compounds will be known or apparent to those skilled in the art and are described in more detail in for example, Remington's Pharmaceutical Science, 17th ed., Mack Publishing Company, Easton, Pa. (1985), and the 18th and 19th editions thereof, which are incorporated herein by reference.

The AC in the composition preferably is formulated in an effective amount. An "effective amount" refers to an amount effective, at dosages and for periods of time necessary, to achieve the desired result, such as treatment of melanoma. A "therapeutically effective amount" means an amount sufficient to influence the therapeutic course of a particular disease state. A therapeutically effective amount is also one in which any toxic or detrimental effects of the agent are outweighed by the therapeutically beneficial effects.

The invention also relates to the AC of the invention, or the pharmaceutical composition of the invention, for use as a medicament, in particular for the treatment of melanoma and other solid cancers in which the human endothelin B receptor is overexpressed, such as glioblastoma, pancreatic cancer, ovarian cancer, bladder cancer, lung cancer, kidney cancer, vulvar cancer or liquid cancers in which the human endothelin B receptor is overexpressed as leukemia, lymphoma, and myeloma.

As used herein, the terms "treat," treating," "treatment," and the like refer to reducing or ameliorating the symptoms of a disorder (e.g., a melanoma) and/or symptoms associated therewith. It will be appreciated that, although not precluded, treating a disorder or condition does not require that the disorder, condition or symptoms associated therewith be completely eliminated.

As used herein "treating" a disease in a subject or "treating" a subject having a disease refers to subjecting the subject to a pharmaceutical treatment, e.g., the administration of a drug, such that the extent of the disease is decreased or prevented. For example, treating results in the reduction of at least one sign or symptom of the disease or condition. Treatment includes (but is not limited to) administration of a composition, such as a pharmaceutical composition, and may be performed either prophylactically, or subsequent to the initiation of a pathologic event. Treatment can require administration of an agent and/ or treatment more than once.

In another embodiment, the present invention also relates to the use of an AC according to the invention or of a pharmaceutical composition according to the invention for the preparation of a drug and/or of a medicament, for the treatment of melanoma and other solid cancers in which the human endothelin B receptor is overexpressed, such as glioblastoma, pancreatic cancer, ovarian cancer, bladder cancer, lung cancer, kidney cancer, vulvar cancer or liquid cancers in which the human endothelin B receptor is overexpressed as leukemia, lymphoma, and myeloma, in a human being.

In a related aspect, the invention provides methods of treating melanoma and other solid cancers in which the human endothelin B receptor is overexpressed, such as glioblastoma, pancreatic cancer, ovarian cancer, bladder cancer, lung cancer, kidney cancer, or vulvar cancer or of treating liquid cancers in which the human endothelin B receptor is overexpressed, such as leukemia, lymphoma, and myeloma, by administering to a human being a therapeutically effective amount of a pharmaceutical composition comprising the AC described herein.

The ACs of the invention can be administered in combination with other treatments directed to treat melanoma as well. In particular, since rendomab-B4 does not inhibit ERK1/2 pathway and this pathway is crucial for melanoma growth, it would be interesting to co-treat the patient with a ERK1/2 pathway inhibitor, or by directly coupling the antibody to such inhibitor.

The dosage of the ACs of the invention administered to a patient is typically about 0.1 mg/kg to about 10 mg/kg of the patient's body weight, e.g., about 0.1, about 0.2, about 0.3, about 0.4, about 0.5, about 0.6, about 0.7, about 0.8, about 0.9, about 1, about 1.5, about 2, about 2.5, about 3, about 3.5, about 4, about 4.5, about 5, about 5.5, about 6, about 6.5, about 7, about 7.5, about 8, about 8.5, about 9, about 9.5, and about 10 mg/kg of the patient's body weight. Preferably, the dosage administered to a patient is between about 1 mg/kg and about 9 mg/kg of the patient's body weight. In other embodiments the dosage of the compositions of the invention is about 0.1, about 0.3, about 1.0 or about 3.0 mg/kg of the patient's body weight.

The ACs of the invention can be administered according to the judgment of the treating physician, e.g., daily, weekly, biweekly or at any other suitable interval, depending upon such factors, for example, as the nature of the ailment, the condition of the patient and half-life of the antibody. In a preferred example, a subject is treated with the antibody or fragment of the invention in the range of between about 0.1 to about 10 mg/kg body weight, one time per week for between about 1 to about 10 weeks, preferably between about 2 to about 8 weeks, more preferably between about 3 to about 7 weeks, and even more preferably for about 4, about 5, or about 6 weeks. In other embodiments, the pharmaceutical compositions of the invention are administered once a day, twice a day, or three times a day. In other embodiments, the pharmaceutical compositions are administered once a week, twice a week, once every two weeks, once a month, once every six weeks, once every two months, twice a year or once per year. It will also be appreciated that the effective dosage of the ACs used for treatment may increase or decrease over the course of a particular treatment.

In a most preferred embodiment, the composition of the invention is administered intravenously over about 30 minutes. In other embodiments, the composition of the invention is administered intravenously over at least about 1 hour, at least about 30 minutes, or at least about 15 minutes.

More generally, for therapeutic applications, the ACs of the invention can be administered to the subject as a bolus or by continuous infusion over a period of time, by intramuscular, intraperitoneal, intracerebrospinal, subcutaneous, intraarticular, intrasynovial, intrathecal, oral, topical, or inhalation routes.

### Diagnostic uses

If the AC carries at least one detection molecule as defined above, preferably two different kinds of detection molecules as defined above, the AC of the invention can be used as an imaging tool, for example as a diagnostic agent. In this case, the AC of the invention do not necessarily comprise a cytotoxic agent (it may contain only detection molecules).

In a particular embodiment, the invention relates to the AC according to the invention, in particular wherein the AC comprises at least one or two different detection molecule(s), for use in pre-, intra- or post-operative imaging, in particular in patients suffering from melanoma or from other solid cancers in which the human endothelin B receptor is overexpressed, such as glioblastoma, pancreatic cancer, ovarian cancer, bladder cancer, lung cancer, kidney cancer, or vulvar cancer or from a liquid cancer in which the human endothelin B receptor is overexpressed, such as leukemia, lymphoma, or myeloma.

Pre-operative imaging encompasses all imaging techniques that may be performed before a surgery to make specific target molecules, cells or tissues visible when diagnosing a certain disease or condition and, optionally, to provide guidance for a surgery. Preoperative imaging may comprise a step of making a tumor visible by PET or SPECT before a surgery is performed by using an AC that comprises an antibody that specifically binds to ET_{B} on the tumor and is conjugated to a detection molecule that comprises a radioisotope.

Intra-operative imaging encompasses all imaging techniques that may be performed during a surgery to make specific target molecules, cells or tissues visible and thus provide guidance for the surgery. In certain embodiments, an AC comprising a near-infrared fluorescent dye may be used to visualize a tumor during surgery by near-infrared fluorescent imaging. Intraoperative imaging allows the surgeon to identify specific tissues, for example tumor tissue, during surgery and thus may allow complete removal of tumor tissue.

Post-operative imaging encompasses all imaging techniques that may be performed after a surgery to make specific target molecules, cells or tissues visible and to evaluate the result of the surgery. Post-operative imaging may be performed similarly as pre-operative surgery.

In certain embodiments, the invention relates to ACs comprising two or more different kinds of detection molecules. For example, the AC may comprise a radioisotope and a near-infrared fluorescent dye. Such an AC may be used for imaging by PET/SPECT and near-infrared fluorescent imaging. The advantage of such AC is that it may be used to visualize the target tissue, for example a tumor before and after a surgery by PET or SPECT. At the same time, the tumor may be visualized during the surgery by near-fluorescent infrared imaging.

Thus, when the ACs of the invention comprise at least one detection molecule, preferably two different kinds of detection molecules as defined above, the present invention generally concerns the use of the AC according to the invention in:
a) a method of diagnosing a tumor by imaging,
b) a surgical treatment method involving tumor resection guided by fluorescence or an isotopic imaging probe,
c) a method of monitoring the effectiveness of an antitumor treatment by imaging, or
d) any combination of methods a), b), and c) (such as a) and b); a) and c); b) and c); or a), b), and c)).

In this case, the AC of the invention do not necessarily comprise a cytotoxic agent (it may contain only detection molecules).

The present invention also concerns a method for detecting tumor cells in a subject by imaging, said method comprising:
a) administering to a subject suspected of having melanoma or another solid cancer in which the human endothelin B receptor is overexpressed, such as glioblastoma, pancreatic cancer, ovarian cancer, bladder cancer, lung cancer, kidney cancer, or vulvar cancer, or suspected of having a liquid cancer in which the human endothelin B receptor is overexpressed, such as leukemia, lymphoma, or myeloma, an AC according to the invention, whose antibody specifically binds to the human endothelin B receptor of the suspected tumor,
b) obtaining one or more images of the suspected organ by imaging, and
c) detecting the presence or absence of tumor cells marked by the conjugate in the image(s) obtained in step b).

The present invention also concerns the use of a conjugate according to the invention in a method for diagnosing a tumor by imaging.

The present invention also concerns a method for determining the tumor load in a patient suffering from a tumor, by imaging, comprising:
a) administering to a subject suffering from melanoma or from another solid cancer in which the human endothelin B receptor is overexpressed, such as glioblastoma, pancreatic cancer, ovarian cancer, bladder cancer, lung cancer, kidney cancer, or vulvar cancer, or suffering from a liquid cancer in which the human endothelin B receptor is overexpressed, such as leukemia, lymphoma, or myeloma, an AC according to the invention, whose antibody specifically binds to the human endothelin B receptor of the patient's tumor,
b) obtaining one or more images of one or more organs of the patient by imaging, and
c) determining the tumor load of the patient from the image(s) obtained in step b).

The detection molecule(s) or those that may become detectable can be chosen based on the type(s) of imaging envisaged:
For use in a method for diagnosing a tumor by isotopic imaging, the AC according to the invention is advantageously conjugated to a radioisotope or a chelating agent (a complex with a radiometal is then formed just before administration to the patient). Notably, for positron emission tomography (PET) or single-photon emission computed tomography (SPECT), the AC according to the invention is advantageously conjugated to a chelating agent (a complex with a gamma-ray or positron emitting radiometal, notably chosen from among ^{99m}Tc, ¹¹¹In, ⁶⁴Cu, ⁶⁸Ga, ⁸⁹Zr, and ⁴⁴Sc, is then formed just before administration to the patient). For Cerenkov Luminescence Imaging (CLI), the AC according to the invention is advantageously conjugated to ⁹⁰Y, ⁶⁸Ga, ²²⁵Ac.

For use in a method for diagnosing a tumor by optical imaging, the AC according to the invention is advantageously conjugated to a chromophore or an affinity molecule (a complex with a chromophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)).

For use in a method for diagnosing a tumor by fluorescence imaging, the AC according to the invention is advantageously conjugated to a fluorophore or an affinity molecule (a complex with a fluorophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)).

The AC according to the invention can also be conjugated to two different kinds of detection molecules (or molecules that may become detectable), allowing its use in two different types of imaging. For example, the AC according to the invention can be conjugated to two detection kinds of molecules (or molecules that may be detected by different imaging techniques), notably:
a1) a gamma-ray or positron emitting radioisotope, covalently bound by means of a chelating agent when the radioisotope is a radiometal, for use in a method for diagnosing a tumor by isotopic imaging (notably by positron emission tomography (PET) or single-photon emission computed tomography (SPECT)) and a fluorophore for use in a method for diagnosing a tumor by fluorescence imaging;
a2) a gamma-ray or positron emitting radioisotope, covalently bound by means of a chelating agent when the radioisotope is a radiometal, for use in a method for diagnosing a tumor by isotopic imaging (notably by positron emission tomography (PET) or single-photon emission computed tomography (SPECT)) and an affinity molecule (a complex with a fluorophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)) for use in a method for diagnosing a tumor by fluorescence imaging;
b1) a gamma-ray or positron emitting radioisotope, covalently bound by means of a chelating agent when the radioisotope is a radiometal, for use in a method for diagnosing a tumor by isotopic imaging (notably by positron emission tomography (PET) or single-photon emission computed tomography (SPECT)) and a chromophore for use in a method for diagnosing a tumor by optical imaging;
b2) a gamma-ray or positron emitting radioisotope, covalently bound by means of a chelating agent when the radioisotope is a radiometal, for use in a method for diagnosing a tumor by isotopic imaging (notably by positron emission tomography (PET) or single-photon emission computed tomography (SPECT)) and an affinity molecule (a complex with a chromophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)) for use in a method for diagnosing a tumor by optical imaging;
c1) a fluorophore or an affinity molecule (a complex with a fluorophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)) for use in a method for diagnosing a tumor by fluorescence imaging and a chromophore for use in a method for diagnosing a tumor by optical imaging; or
c2) a fluorophore for use in a method for diagnosing a tumor by fluorescence imaging and an affinity molecule (a complex with a fluorophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)) for use in a method for diagnosing a tumor by optical imaging.

Among the conjugates with two detection molecules or molecules that may be detected by different imaging techniques described above, conjugates a1) and b1) are preferred.

Alternatively, the AC according to the invention can be conjugated to two kinds of detection molecules (or molecules susceptible to being detected) by the same imaging technique (which increases the signal and therefore the sensitivity of detection), notably:
a) two gamma-ray or positron emitting radioisotopes (different or preferably identical), covalently bound by means of chelating agents (different or preferably identical) when the radioisotopes are radiometals, for use in a method for diagnosing a tumor by isotopic imaging (notably by positron emission tomography (PET) or single-photon emission computed tomography (SPECT)),
b) two fluorophores (different or preferably identical) for use in a method for diagnosing a tumor by fluorescence imaging,
c) two chromophores (different or preferably identical) for use in a method for diagnosing a tumor by optical imaging, or
d) two affinity molecules (different or preferably identical) for use in a method for diagnosing a tumor by optical or fluorescence imaging.

Among the conjugates with two detection molecules (or molecules susceptible to being detected) by the same imaging technique described above, the conjugates a) (two gamma-ray or positron emitting radioisotopes (different or preferably identical) or two chelating agents (different or preferably identical)) and b) (two fluorophores (different or preferably identical)) are preferred.

Alternatively, the AC according to the invention can also be conjugated to one detection molecule or one molecule susceptible to becoming detectable and one pharmacomodulatory molecule, which allows the modification of pharmacokinetic properties of the diagnostic conjugate.

In this embodiment, the AC according to the invention can also be conjugated to:
a) a pharmacomodulatory molecule and a gamma-ray emitting or positron radioisotope, covalently bound by means of a chelating agent when the radioisotope is a radiometal, for use in a method for diagnosing a tumor by isotopic imaging (notably by positron emission tomography (PET) or single-photon emission computed tomography (SPECT));
b) a pharmacomodulatory molecule and a fluorophore for use in a method for diagnosing a tumor by fluorescence imaging;
c) a pharmacomodulatory molecule and a chromophore for use in a method for diagnosing a tumor by optical imaging;
d) a pharmacomodulatory molecule and an affinity molecule (a complex with a fluorophore conjugated to a binding partner of the affinity molecule is then formed before administration to the patient, the couple (affinity molecule/binding partner) being advantageously chosen from (biotin/avidin), (biotin/streptavidin), (avidin/biotin), and (streptavidin/biotin)) for use in a method for diagnosing a tumor by optical or fluorescence imaging.

Among the conjugates with one detection molecule or one molecule susceptible to becoming detectable and one pharmacomodulatory molecule described above, the conjugates a) (a pharmacomodulatory molecule and a gamma-ray emitting or positron radioisotope or a chelating agent) and b) (a pharmacomodulatory molecule and a fluorophore) are preferred.

The invention also concerns the AC according to the invention, for its use in a surgical treatment method involving tumor resection guided by fluorescence or an isotopic imaging probe. The present invention also concerns a method of surgical treatment involving tumor resection guided by fluorescence or an isotopic imaging probe, comprising:
a) administering to a subject suffering from melanoma or another solid cancer in which the human endothelin B receptor is overexpressed, such as glioblastoma, pancreatic cancer, ovarian cancer, bladder cancer, lung cancer, kidney cancer, or vulvar cancer, an AC according to the invention, whose antibody specifically binds to the human endothelin B receptor of the patient tumor,
b) obtaining real-time images of the tumor marked by the conjugate through imaging, and surgically resecting the entire tumor using the real-time images obtained.

In this application, the AC according to the invention is conjugated to:
a1) a fluorophore for use in a surgical treatment method involving tumor resection guided by fluorescence;
a2) an affinity molecule (a complex with a fluorophore conjugated to a binding partner of the affinity molecule is then formed before administration to the patient, the couple (affinity molecule/binding partner) being advantageously chosen from among (biotin/avidin), (biotin/streptavidin), (avidin/biotin), and (streptavidin/biotin)) for use in a surgical treatment method involving tumor resection guided by fluorescence imaging;
b1) a chelating agent (in this case, a complex with a radiometal, which directly or indirectly emits gamma rays, is formed just before administration to the patient) for use in a surgical treatment method involving tumor resection guided by an isotopic imaging probe, notably by single-photon emission computed tomography (SPECT) imaging probe (in that case, the radiometal is a direct gamma-ray emitter, such as ^{99m}Tc and ¹¹¹In) or by positron emission tomography (PET) (in that case, the radiometal is an indirect gamma-ray emitter, such as ⁶⁴Cu, ⁶⁸Ga, ⁸⁹Zr, and ⁴⁴Sc), with a preference for use in a surgical treatment method involving tumor resection guided by a single-photon emission computed tomography (SPECT) imaging probe (in that case, the radiometal is a direct gamma-ray emitter, such as ^{99m}Tc and ¹¹¹In);
b2) a direct or indirect gamma-ray emitting radioisotope (such as ¹⁸F) for use in a surgical treatment method involving tumor resection guided by an isotopic imaging probe;
c1) a molecule defined in a1) and a molecule defined in b2), thus allowing its use in a surgical treatment method involving tumor resection guided by fluorescence or by an isotopic imaging probe; or
c2) a molecule defined in a2) and a molecule defined in b2), thus allowing its use in a surgical treatment method involving tumor resection guided by fluorescence or by an isotopic imaging probe.

In this method, conjugates a1), b1), and c1) are preferred.

The invention also concerns the AC according to the invention, for its use in a method of imaging-based monitoring of the effectiveness of an antitumor treatment. The present invention also concerns a method for treating a subject suffering from melanoma or from another cancer in which the human endothelin B receptor is overexpressed, for example glioblastoma, pancreatic cancer, ovarian cancer, bladder cancer, lung cancer, kidney cancer, or vulvar cancer, said comprising:
a) determining the patient's tumor load using the tumor load determination method according to the invention before or concomitantly with the beginning of the antitumor treatment administration,
b) administering the antitumor treatment to the patient,
c) determining the patient's tumor load using the tumor load determination method according to the invention after the beginning of the antitumor treatment administration,
d) comparing the tumor loads determined in steps a) and c), and
e) administering an antitumor treatment to the patient, where:
   - if the tumor load determined in step c) is equal to or lower than that determined in step a), then the antitumor treatment administered in step e) is the same as in step b);
   - if the tumor load determined in step c) is higher than that determined in step a), then the antitumor treatment administered in step e) is different from that administered in step b).

The detection molecule or molecule that may become detectable will be chosen based on the type(s) of imaging envisaged, as described above.

To determine the effectiveness of an antitumor treatment, the AC according to the invention is advantageously used:
a) before or concomitantly with the beginning of the antitumor treatment administration, and
b) at least once after the beginning of the antitumor treatment administration,
and the tumor load detected after the beginning of the antitumor treatment administration is compared to that detected before or concomitantly with the beginning of the antitumor treatment administration.

If the tumor load has decreased or remains stable after the beginning of the antitumor treatment administration, then the antitumor treatment is considered effective. If the tumor load has increased after the beginning of the antitumor treatment administration, then the antitumor treatment is considered ineffective.

### Definitions

By "a" or "an," we mean "one or more". In other words, when we use "a" or "an" in relation to a characteristic, it covers both implementations with a single instance of the characteristic of interest and those with multiple instances of the characteristic of interest. For example, an antibody fragment that includes a mutated heavy chain variable domain can include a single mutated heavy chain variable domain (such as in an Fv or scFv fragment) or multiple mutated heavy chain variable domains (such as in a Fab fragment, a diabody, tribody, or tetrabody).

By "one molecule", it is not herein intended to limit the number of molecules that are actually coupled to the antibody of the invention, but to insist on the fact that the AC of the invention comprises one kind of detection molecule (i.e., one or several detection molecules of this kind, coupled to each antibody molecule) or one kind of cytotoxic molecule (i.e., one or several cytotoxic molecules of this kind coupled to each antibody molecule). In a preferred embodiment, the antibody of the invention is coupled to one or several identical detection molecules, and/or to one or several identical cytotoxic molecules). As the antibody of the invention preferably contains two chains each containing a reactive amino acids where the molecules can be bound, the antibody of the invention is preferably coupled to two identical detection molecules or to two identical cytotoxic molecules. In a more preferred embodiment, the antibody of the invention is preferably coupled to two identical detection molecules and to two identical cytotoxic molecules. The antibody of the invention can also be coupled to four detection molecules of two sorts or to four cytotoxic molecules of two sorts. If the antibody of the invention contains more than two coupling sites for the functional molecules, more combinations and numbers can be foreseen.

In this document, when used to define products, compositions, and methods, the terms "comprising" (and any form of "comprising," such as "comprises" and "comprising"), "having" (and any form of "having," such as "has" and "having"), "including" (and any form of "including," such as "includes" and "including"), or "containing" (and any form of "containing," such as "contains" and "containing") are open-ended and do not exclude additional elements or method steps not mentioned. Thus, a polypeptide "comprises" an amino acid sequence when the amino acid sequence is part of the final amino acid sequence of the polypeptide. Such a polypeptide can have up to several hundred additional amino acid residues. By "consisting essentially of" or "consisting essentially of," we mean the exclusion of other components or steps of any essential importance. Thus, a polypeptide "consists essentially of" an amino acid sequence when such an amino acid sequence is present with possibly only a few additional amino acid residues (e.g., a peptide of at most 20 amino acids, such as a His6 tag, may additionally be present). "Consisting of' or "consisting of" means excluding more than trace amounts of other components or steps. For example, a polypeptide "consists of' an amino acid sequence when the polypeptide contains no other amino acids than the indicated amino acid sequence.

The term "glycosylation site" refers to an amino acid residue that is recognized by a mammalian cell as a location for the attachment of sugar residues. Amino acid residues to which carbohydrates, such as oligosaccharides, are attached are usually asparagine (N-linkage), serine (O-linkage), and threonine (O-linkage) residues. The specific sites of attachment usually have a characteristic sequence of amino acids, referred to as a "glycosylation site sequence." The glycosylation site sequence for N-linked glycosylation is : -Asn-X-Ser- or -Asn-X-Thr-, where X can be any of the conventional amino acids, other than proline. The Fc region of human IgG has two glycosylation sites, one in each of the CH2 domains. The glycosylation that occurs at the glycosylation site in the CH2 domain of human IgG is N-linked glycosylation at the asparagine at position 297 (Asn 297).

### FIGURE LEGENDS

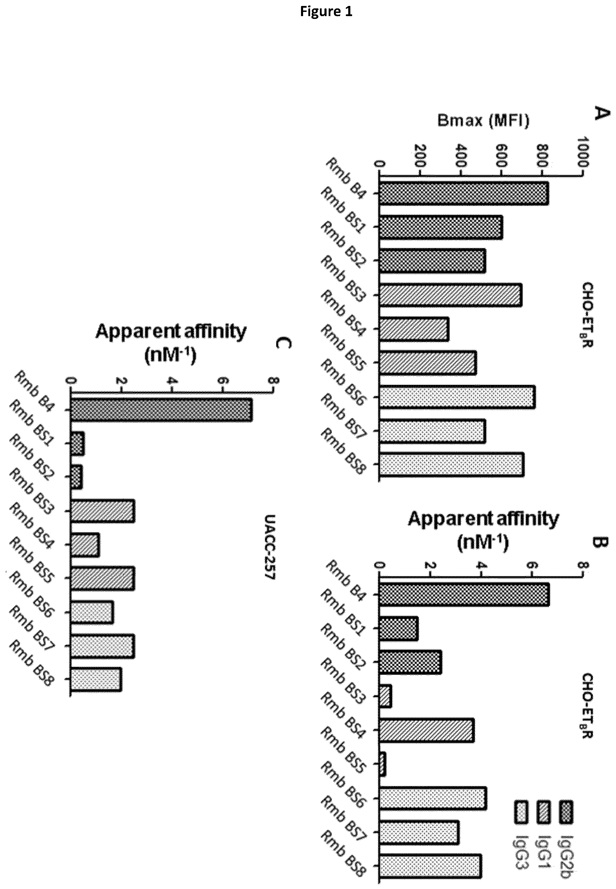

**Figure 1** shows the binding properties of anti-human ET_{B} specific monoclonal antibodies. To evaluate maximal binding (A) and apparent affinities (B, C), CHO and UACC-257 cells expressing hETs were incubated for 24 h at 4°C with increasing concentrations of ET_{B} directed antibodies. Binding of anti-ET_{B} antibodies were revealed using R-PE-labeled anti-mouse antibody and quantified by flow cytometry. Each antibody's maximal binding (Bmax) and apparent affinity (1/apparent K_{d}) were determined using GraphPad Prism software.

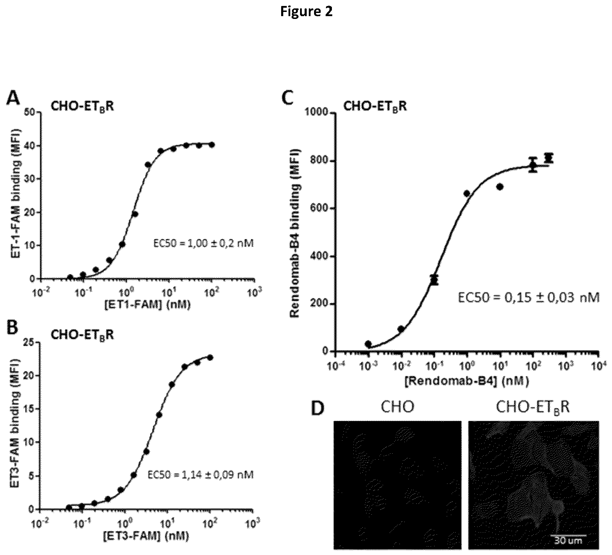

**Figure 2** shows that rendomab-B4 specifically recognizes with high affinity human ET_{B} overexpressed in CHO cell line. To check human ET_{B} expression on CHO-ET_{B} surface, cells were incubated with increasing concentrations of ET-1-FAM (A) and ET-3-FAM (B) for 24 h at 4°C in the dark. To determine rendomab-B4 affinity, increasing concentrations of the monoclonal antibody were incubated with CHO-ET_{B} cells for 24 h at 4°C (C). Rendomab-B4 binding was revealed using R-PE-labeled anti-mouse antibody. The binding of labeled-peptides and monoclonal antibody were measured on a GUAVA flow cytometer and resulting curves (A, Band C), which correspond to mean fluorescence intensity (MFI) as a function of ligand concentrations, were plotted and fitted using GraphPad Prism software. K_{d} or apparent K_{d} (K_{d}*) corresponding to half maximal effective concentration values correspond to the mean § s.d. of three (A and B) or 5 (C) independent experiments. (D) CHO and CHO-ET_{B} cells were fixed and incubated with 100nM of rendomab-B4, followed by Cy3-conjugated anti-mouse IgG antibody incubation.
**Figure 3** shows that rendomab-B4 does not compete with ET-1 and ET-3 for binding CHO-ET_{B}. CHO-ET_{B} cells were incubated with 10 nM of ET-1-FAM (A) or ET-3-FAM (B) and simultaneously with increasing concentrations of isotype control antibody, rendomab-B4, selective receptor antagonists (BQ123 or BQ788) and the corresponding unlabelled endothelin (ET-1 or ET-3). (C) Cells were incubated simultaneously with 100 nM of rendomab-B4 and with increasing concentrations of endothelin (ET-1 or ET-3) or control peptide. Rendomab-B4 binding on cell surface was detected with a R-PE-labeled anti-mouse antibody. The fluorescence (A, B and C) was quantified by flow cytometry. Inhibition binding curves, corresponding to MFI relative to competitor concentration, were plotted and fitted with GraphPad Prism software. Two independent experiments were performed.
**Figure 4** shows that rendomab-B4 specifically recognizes ET_{B} in melanoma: Binding properties. (A) Human melanoma cell lines (UACC-257, WM-266-4 and SLM8), HEK293T and HUVEC were incubated with increasing concentrations of rendomab-B4. Rendomab-B4 binding was evaluated using R-PE-labeled anti-mouse antibody followed by flow cytometry analysis. The results are representative of 3 independent experiments. (B) To control human ET_{B} expression at the surface of UACC-257 cells, the cells were incubated with varying concentrations of ET-3-FAM and fluorescence was measured by flow cytometry. Three independent experiments were performed. (C) UACC-257 melanoma cells were simultaneously incubated with 10 nM of ET-3-FAM and increasing concentrations of isotype control antibody, rendomab-B4, selective antagonists of ET_{A} (BQ123) or ET_{B}(BQ788) or unlabelled ligand ET-3. (D) Differential rendomab mAbs binding. The rendomab B49 and rendomab B1 were incubated at different concentrations on A-375 and WM-266-4 human melanoma cells. Rendomab B49 exhibited binding curves with a similar apparent subnanomolar Kd as rendomab B4. Conversely, rendomab B1 did not show binding curves on these melanoma cells. The fluorescence was quantified by flow cytometry. All these curves were plotted and fitted using GraphPad Prism software.
**Figure 5** shows that rendomab-B4 is internalized in UACC-257 cells. Flow cytometry experiments were performed to study the internalization of ET_{B}(A) and of rendomab-B4 itself (B). (A) Living, adherent UACC-257 cells were incubated at 4°C (green curve) or 37°C (pink curve) in medium in the presence of 50 nM ET-3. After washing, cells were detached and incubated for 2 hours at 4°C with rendomab-B4 in order to evaluate ET_{B} amount at the surface of the cells. Cells were then incubated with a fluorescent secondary antibody and the fluorescence was measured using a FACScalibur cytometer. Black curve corresponds to basal fluorescence. (B) UACC-257 living cells were incubated for 3hours at 4°C(green curve) with 100nM rendomab-B4.Cells were incubated for 3 hours at 37° C with 100nM rendomab-B4 prior to 1 additional hour incubation at 37°C with 50 nM ET-3 (blue curve) or not (pink curve). Cells were then detached and remaining antibodies at cell surface were detected using secondary antibody. The fluorescence was quantified by flow cytometry. (Cand D) Living UACC-257 cells were incubated for 2 h at 4°C(C) or 37°C(D) with rendomab-B4, fixed and incubated in Cy3-anti-mouse antibody (red). Green labeling in (E) corresponds to early endosomal antigen 1 (EEA1) staining. Merged image is represented in (F).
**Figure 6** shows that rendomab-B4 inhibits PLC but not ERK response due to ET_{B} receptor activation in UACC. (A) [³H]inositol-labeled UACC-257 cells were treated or not for 2 h with 150 nM of control isotype antibody or Rendomab-B4 before stimulation for 30 min in the presence or the absence of 50 nM ET-1 or ET-3. Total [³H]InsPs amount was determined as described in materials and methods. Results are expressed as percent of InsPs production induced by ET-1 in the absence of antibody and are means ± SEM of 6 independent experiments, each performed in duplicate. (B) Cells were treated for 2 h with or without 150 nM of control isotype antibody or rendomab-B4 before the addition of 10 nM ET-1 or ET-3. After a 10 min incubation, cells were lysed and total proteins were analyzed by 10%SDS/ PAGE followed by immunoblotting with anti-active phosphorylated ERK1/2 (pERK) antibody and anti-total ERK2 (ERK). pERK and ERK signals were quantified (C), and the levels of phosphorylated ERK1/2 were normalized with respect to total ERK2 amount in the corresponding sample. Results were expressed as percent of ET-1 stimulation without antibody treatment (100%). Values are the means ± SEM of 3 independent experiments performed in duplicate.
**Figure 7** shows that rendomab-B4 inhibits melanoma cell migration induced by endothelin. UACC-257 (150 nM) seeded in culture inserts were treated for 2 h with or without 150nM of rendomab-B4 or control isotype antibody before the addition of 10 nM ET-1. After 20 h of incubation, cells were stained with crystal violet and counted from 3 randomly selected images per insert. (A) Images of UACC-257 cells on the lower side of the insert membranes. (B) Quantification of the number of cells present on the lower side of the insert membranes. Values are the means ± SEM of 3 independent experiments.
**Figure 8** shows (A) the structure of a preferred trivalent moiety that can be used to attach the molecules of interest to the antibody part of the AC of the invention. If R is NH2, then an enzymatic conjugation thanks to the microbial transglutaminase mTGase can be used. If R is maleimide, conjugation can be performed on the thiol groups of the cysteine residues of the antibody. If R is an isothiocyanate or squaramate group, conjugation can be performed on the lysine residues of the antibody. R1 and R2 can be any chelating agent, coupling agent or functional molecules of interest disclosed herein. A preferred moiety (B) is provided, with the lysine moiety being coupled to DBCO (left) and DOTAGA (right) as coupling and chelating agents respectively.

### EXAMPLES

The examples that follow are merely exemplary of the scope of this invention and content of this disclosure. One skilled in the art can devise and construct numerous modifications to the examples listed below without departing from the scope of this invention.

### 1. Material and methods

### Animals

Six-week-old female C57BL/6 mice (Elevage Janvier) were kept in a specific pathogen-free animal facility. All animal experiments complied with French animal experimentation regulations.

### Plasmids

The cDNA clone of the human ET_{B} was subcloned in pcDNA3.1 vector (Life Technologies). The integrity of the con- struct was confirmed by sequencing.

### Cell culture and transfection

CHO-K1 cells (ECACC) were cultured in Ham-F12 medium, HEK293T (EACC) and human umbilical vascular endothelial cells (HUVEC, ATCC) in DMEM medium. The melanoma cell line UACC-257 (NCI-60 cell collection) was cultured in RPMI-1640. Melanoma cell lines WM-266-4 (ECACC) and SLM8 were cultured in DMEM: Ham-F12 medium (1:1). All media were supplemented with 10% fetal calf serum, 1 mM pyruvate, 1% nonessential amino acids, 2 mM glutamine, 100 U/mL penicillin and 100 µg/mL streptomycin. Eker rat uterine leiomyoma cells (ELT-3) were cultured in DF8 medium supplemented with 10% fetal calf serum. Cells were maintained at 37 C in a humidified atmosphere of 5% CO₂. All media and cell culture supplements were from Life Technologies. The ET_{B} expression vector was transfected and stably overexpressed in CHO and HEK cells using FUGENE HD reagent (Roche Diagnostics). Stably transfected cells expressing human ET_{B} or ET_{A} were termed CHO-ET_{B}, CHO-ET_{A} or HEK-ET_{B}, and they were always cultured in the presence of 1 mg/mL G1418.

### DNA immunization protocol and production of monoclonal antibodies

MAb production, by DNA immunizations, was performed as previously described in Allard B. et al, 2011 ; collected splenocytes of the 2 best- responding mice were fused to NS1 mouse myeloma cells. Hybridoma supernatants were screened for production of anti- ET_{B} specific antibodies by a living cell-based ELISA test, using untransfected CHO cells and CHO-ET_{B} cells as targets, prior to confirmation of specificity and reactivity of antibodies by flow cytometry. After subcloning by limiting dilutions, antibodies were isotyped using a mouse immunoglobulin isotyping kit according to the manufacturer (Pierce) instructions and purified by affinity chromatography on Protein A-Sepharose (Millipore).

### Flow cytometry analysis

### Affinity determination

For affinity measurements, saturation binding experiments were performed with stably transfected CHO-ET_{B}, melanoma cell lines (UACC-257, WM266-4 and SLM8), HEK293T and HUVEC. Collected cells were seeded (100,000 cells/well) in V-shaped 96-well plates. Plates were centrifuged, supernatant was discarded and cells were incubated overnight at 4°C with 100 µL of D-phosphate-buffered saline (PBS) supplemented with 0.1% BSA and 5% normal goat serum (NGS, Life Technologies) and containing increasing concentrations of rendomab- B4. After two washes with 150 µL of ice-cold D-PBS - 0.1% BSA - 1% NGS, cells were incubated for 2 h at 4°C in the dark with R-phycoerythrin (R-PE)-conjugated AffiniPure goat anti-mouse IgG (H⁺L) (Jackson ImmunoResearch). After two washes, cells were re-suspended in 100 rrL of D-PBS and their fluorescence was measured using a GUAVA flow cytometer (Guava Easycyte Plus, Millipore). Mean fluorescence intensity (MFI) of samples was then calculated. To control ET_{B} expression at the cell surface, increasing concentrations of fluorescein-labeled ET-1 or ET-3 (ET-1-FAM or ET-3-FAM, Phoenix Pharmaceuticals) were used.

### Competition experiments

For competition tests, CHO-ET_{B} or melanoma cells (UACC- 257) were treated according to 2 different protocols: (i) cells were co-incubated with 10 nM ET-1-FAM or ET-3-FAM and varying concentrations of different competitors (rendomab-B4, control isotype antibody, ET-1, ET-3, selective ET_{A} receptor antagonist (BQ123) or selective ET_{B} receptor antagonist (BQ788); and (ii) cells were co-incubated with fixed rendomab- B4 concentration (10 nM) and varying concentrations of competitor peptides ET-1, ET-3. To reach equilibrium, cells were incubated overnight at 4°C. For type (i) experiments, cells were washed twice before measuring the fluorescence. For type (ii) experiments, after 2 washes, cells were incubated for 2 h with (R-PE)-conjugated secondary antibody. The MFI of the cells was measured using a GUAVA cytometer.

### Receptor and rendomab-B4 internalization

To study receptor and antibody internalization in UACC-257 cell line, 300,000 cells were used per assay. The internalization of the receptor due to ET was evaluated by incubating living adherent cells for 1 h at 4°C or 37°C with 50 nM ET-3. After detachment, cells were incubated for 2 h at 4°C in D-PBS - 0.1% BSA - 5%NGS with 100 nM rendomab-B4, washed twice and were incubated for 2 h at 4°C with (R-PE)-conjugated AffiniPure goat anti-mouse IgG (H⁺L). Cells were washed twice and the fluorescence was analyzed. For antibody internalization experiments, living adherent cells were incubated for 3 h at 4°C or 37°C with either 100 nM rendomab-B4 or control isotype antibody. Cells were incubated for one additional hour with or without 50 nM ET-3. Then, cells were detached and incubated for 2 h at 4°C with (R-PE)- conjugated AffiniPure goat anti-mouse IgG (H⁺L) and were washed twice again. For these 2 kinds of experiments, cell fluorescence was finally assayed using a FACSCalibur flow cytometer.

### Microscopy analysis

### Wide-field microscopy analysis

Wide-field microscopy analysis was performed on CHO and CHO-ET_{B} cell lines seeded on glass coverslips at the density of 15,000 cells per cm2. After 48 h, cells were washed in PBS and fixed for 15 min in 4% paraformaldehyde (PFA) at room temperature. Cells were then incubated for 10 min in 50 mM NH₄Cl and washed once with PBS. Cells were then incubated at room temperature for 1 hour in the presence of 100 nM of rendomab-B4 in PBS-1% BSA. This incubation is performed in the absence of detergent in order to only detect membrane receptors at the cell surface. After three successive washes with PBS, cells were incubated for 1 h with PBS - 1% BSA containing Cy3-conjugated anti-mouse IgG antibody (Jackson ImmunoResearch). After three washes, cells were mounted on slides with Fluoroshield containing DAPI (Sigma-Aldrich). Images were taken using a fluorescence microscope (Zeiss Axiophot II), at 40x magnification.

### Confocal microscopy analysis

Confocal microscopy analysis was performed on UACC-257 cells seeded on glass coverslips at a density of 30,000 cells per cm2. For internalization experiments, living cells were incubated at 4 or 37°C for 1 h with 100 nM of rendomab-B4 before fixation with 4% PFA. To stain the endosomal compartment, cells were permeabilized by treatment for 15 min with PBS -1% BSA - 0.2% Triton X-100 before incubation with an anti-EEA1 antibody (Millipore, France). After three washes, cells were incubated in PBS -1% BSA - 0.2% Triton X-100 containing Cy3-conjugated anti-mouse IgG antibody and FITC-conjugated anti-rabbit antibody. Images were taken using a confocal microscope (Zeiss LSM510), at 63x magnification.

### Peptide synthesis and ET_{B}-binding epitope mapping

The entire extracellular amino acid sequence of the ET_{B} receptor was synthesized on a cellulose membrane using the previously described SPOT technique (Laune D. et al, 2002), laying down overlapping 12-mer peptides, frameshifted by one residue. Rendomab-B4 epitope mapping was performed according to a protocol previously described (Allard B. et al, 2011), incubating the membrane in 1 mg/mL of rendomab-B4 for 90 min at room temperature. The hybridization was revealed using an anti-mouse IgG labeled with alkaline phosphatase (Sigma-Aldrich). ImageJ software was used to quantify the signal obtained for each spot. Peptides were considered as antigenically relevant if they were part of a consecutive series of reactive spots, presenting at least one signal peak times higher than the background (Laune D. et al, 2002).

### Cloning of GFP-tagged rat and human ET_{B}

Rat ET_{B} cDNA was cloned by RT-PCR technique from rat ELT3 cells, which express high level of ET_{B} (Raymond MN et al, 2009). Total RNA from these cells was prepared using NucleoSpin ^{®} RNAII (Macherey-Nagel) according to the manufacture's protocol.

The reverse transcription reaction was performed with 5 µg of total RNA using 200 units of Moloney Murine Leukemia Virus (M-MLV)-reverse transcriptase, 200 µM deoxynucleoside triphosphates (dNTPs), and 10 µM random hexamer primers. PCR primers for amplification of rat EBT (rET_{B}) were based on the GenBank sequence (NM_017333). The primers span the start codon and introduced a modified codon in place of the stop codon and contain Hindlll and BamHl sites (italics):
50-GC*AAGCTT*ATGCAATCGTCCGCAAGCC-30 (Forward primer, SEQ ID NO:20) and
50-GC*GGATCC*ACAGATGAGCTGTATTTATTGCTGG -30 (Reverse primer, SEQ ID NO:21).

For hET_{B} cloning, hET_{B} cDNA was amplified by PCR from pCDNA3.1-ET_{B} plasmid (described above) with PCR primers based on the GenBank sequence (NM_000115.3). As for rET_{B} cloning, the primers span the start codon and introduced a modified codon in place of the stop codon and contain Hindlll and BamHl sites (italics):
50-GC*AAGCTT*ATGCAGCCG CCTCCAAGTC-30 (Forward primer, SEQ ID NO:20) and
50-GC*GGATCC*ACAGATGAGCTGT ATTTATTACTGG-30 (Reverse primer, SEQ ID NO:21).

The PCR reactions were performed, in a thermal cycler (icycler; Bio-Rad) with 3 ml of the RT reaction (rET_{B}) or 10 ng pCDNA-ET_{B} plasmid (hET_{B}) using the Phusion^{®} high fidelity DNA Polymerase according to the manufacturer's protocol.

The amplified condition was 98°C for 30 s followed by 30 cycles of 98°C for 10 s, 70°C for 30 s, 72°C for 45 s, and a final extension at 72°C for 10 min. The PCR products were digested by BamHl and Hindlll restriction enzymes and ligated into pEGFP-N1 (Clonetech), using T4 DNA ligase according to the manufacturer's indication. Resulting plasmids were named phET_{B}-GFP and prET_{B}-GFP, and their sequences were verified by sequencing (Eurofins MWG Operon).

### Immunoprecipitation of ET_{B}

CHO cells seeded in 24-well plates were transfected with phET_{B}-GFP or prET_{B}-GFP plasmids using Lipofectamine LTX (Life Technologies) according to the manufacturer's instructions. 24 h after transfection, cells were washed with PBS and lysed in 200 µL of ice cold lysis buffer containing 10 mM Tris/ Cl pH 7.5; 150 mM NaCl; 0.5 mM EDTA; 1% Triton X-100. Lysates were centrifuged for 5 min at 15,000 × g and the supernatants diluted to 1 mL with lysis buffer without Triton X-100.

Antibodies, 4 µg of rendomab-B4 or 4 µL of GFP-Trap beads (Chromotek), were added and the samples were incubated on a wheel for 4 h at 4 C. When rendomab-B4 was used, 20 µL G-protein conjugated beads (Santa Cruz) were added after the 2 first hours of incubation. At the end of the incubations, samples were washed 4 times with 1 mL ice cold lysis buffer without detergent. Immunoprecipitated samples were then analyzed by protein gel blot using an anti-GFP antibody (Cell Signaling Technologies) and a secondary anti-rabbit IgG antibody conjugated to HRP (Cell Signaling Technologies).

### Measurement of PLC activity

Confluent UACC-257 cells seeded in 12-well plates were serum starved for one night in MEM in the presence of 10 µCi/mL Myo-[2-³H]inositol (16 Ci/mmol; PerkinElmer). The cells were washed 3 times with MEM and then incubated at 37°C for 2 h in 800 µl of MEM in the presence of 150 nM rendomab-B4 or control isotype antibody. Cells were then exposed to the agonists ET-1 or ET-3 (50 nM) in the presence of 10 mM LiCI (added 20 min before the agonists). Total inositol phosphates (InsPs) produced by PLC were quantified as previously described in Raymond MN et al, 2009. Results were expressed as the mean
± SEM of at least 3 independent experiments performed in duplicate.

### Western blot analysis of phosphorylated ERK1/2

Confluent UACC-257 cells seeded in 12-well plates were serum starved for one night in RPMI and then incubated at 37 C for h in 800 µl of MEM in the presence of 150 nM rendomab-B4 or control isotype antibody. Cells were then exposed to the agonists ET-1 or ET-3 (50 nM) for 5 min and then lysed in 50 ml lysis buffer (50 mM HEPES (pH 7.4), 150 mM NaCl, 100 mM NaF, 10% glycerol, 10 mM Na₄P₂O₇, 200 mM Na₃VO₄, 10 mM EDTA, 1% Triton X-100, 10 mg/mL aprotinin and leupeptin). Detergent-extracted proteins were analyzed by western blot technique using mouse monoclonal anti-active phosphorylated ERK1/2 (Cell Signaling Technology) and rabbit polyclonal anti-ERK2 (Santa Cruz Biotechnology) antibodies (1:5000 each). The membranes were then incubated with antirabbit IgG antibody conjugated to IRDye 800CW (LICOR Biosciences) and antimouse IgG antibody conjugated to AlexaFluor 680 (Life technologies) for 1 h at 37°C. Protein bands were detected and quantified on a 2color Odyssey Infrared Imaging System (LICOR Biosciences).

### Migration assay

UACC-257 cells were seeded within cell culture inserts (Falcon PET membrane with 8 mm porosity) placed in a 24-well plate (30,000 cells/insert). After 8 h of culture, media were removed and the cells were incubated for 2 h in RPMI medium without FCS, in presence or absence of 150 nM rendomab-B4 or 150 nM antibody control. Cells were then stimulated or not by addition of 50 nM ET-1 in the 2 compartments. After 20 h, inserts were removed and the cells were stained with crystal violet (0.1% in 20% ethanol). After scrapping off non-migrated cells, the inserts were observed on an Axiophot II Zeiss microscope and images were taken at low magnification. The mean number of cells present on each insert was determined from 3 randomly selected images.

### Chimerization of the RB4 antibody to produce the chimeric antibody xiRB4

A chimeric version of RB4 called xiRB4 was generated. Heavy and light chains encoding IgG-xiRB4 were subcloned into the pTT5 expression plasmid containing either human IgG1 or human Kappa sequences. Vectors were co-transfected into ExpiCHO-S cells (ThermoFisher Scientific) with the ExpiCHO Expression System Kit (ThermoFisher Scientific) according to the manufacturer's instructions (MAN0014337 ThermoFisher Scientific). Day 12 post-transfection, supernatant was clarified by centrifuging at 4000-5000 × g for 30 min at 4 °C and filtered through a 0.22 µm filter. xiRB4 was purified on a HiTrap Protein A HP column (GE HealthCare). Following elution, antibody solutions were dialyzed with Slide-A-Lyzer^{™}G2 Dialysis Cassette (ThermoFisher Scientific) into 1 L of phosphate-buffered saline (PBS).

### Production of the DBCO-Lys(NH₂)-DOTAGA probe

Boc-L-Lys(Fmoc)-OH (1 equiv., 107 µmol, 50 mg), N,N-Diisopropylethylamine (2 equiv., 213 µmol, 37 µL) and N,N,N',N'-Tetramethyl-O-(N-succinimidyl)uroniumtetrafluoroborate (1.6 equiv., 171 µmol, 51.5 mg) in 500 µL of dry DMSO were stirred at room temperature for 30 min to obtain an orange solution. NH₂-DOTA-GA(tBu)₄ (1 equiv., 107 µmol, 135 mg) and N,N-Diisopropylethylamine (10 equiv., 1067 µmol, 186 µL) were then added to the solution. The reaction mixture was stirred at room temperature for 3 hours and purified on semi-preparative HPLC on a BetaBasic-18 column (A: H2O 0.1% TFA, B: MeCN 0.1% TFA; with the following gradient program: 2% of B for 8 min, 2 to 30% in 5 min, 30 % to 70% of B in 30 min, at a flow rate of 20 mL/min) to obtain a white solid after lyophilization (m = 65.2 mg, y = 52%).

RP-HPLC-MS: tr = 4 min, m/z calculated for C₆₃H₁₀₀N₈O₁₄ [M+H]+ 1193.7, found 1193.9.

Boc-L-Lys(Fmoc)-DOTAGA (1equiv., 23.1 µmοl) was dissolved into 500 µl of CH₂Cl₂ and 750 µl of TFA and stirred at room temperature for 2h30. After concentration under vacuum, the resulting solution was diluted in 200 µl of dry DMF before the addition of DBCO-NHS (1.05 equiv., 24 µmol, 10 mg) and N,N-Diisopropylethylamine (17 equiv., 391 µmol, 38.4 µL). The reaction mixture was then stirred at room temperature for 2 hours. Finally, 100 µl of diethyl amine solution in DMF (2/8) were added and stirred at room temperature for 1 hour. After concentration under vacuum, the mixture was purified by semi-preparative HPLC on a BetaBasic-18 column (A: H₂O 0.1% TFA, B: MeCN 0.1% TFA; with the following gradient program: 5% of B for 5 min, 5 to 25% in 10 min, 25 % to 35% of B in 20 min, at a flow rate of 20 mL/min) to afford a beige powder (m = 6.8 mg, y = 30%).

RP-HPLC-MS: tᵣ = 3.0 min, m/z calculated for C₄₈H₆₇N₉O₁₂ [M+H]⁺ 962.5 found 962.6.

### Obtaining xiRB4_IgG1-MMAE-DOTAGA in Two Steps

### Step 1): Conjugation of xiRB4_lgG1 with the DBCO-Lys(NH2)-DOTAGA Probe:

To a solution of xiRB4_lgG1, 3U of mTGase/mg of protein and 15 equivalents of DBCO-Lys(NH₂)-DOTAGA probe are added. The antibody concentration is then adjusted to 5mg/ml by adding PBS. The reaction medium is stirred at 37°C overnight. The xiRB4_lgG1_DBCO-DOTAGA conjugate is then purified by size exclusion chromatography (Sephadex G-25 M, PD-10 column, Cytiva) and concentrated using a centrifugal filter with a molecular weight cut-off of 50kDa (AmiconTM Ultra 2 mL Centrifugal Filtration Units, Millipore Corp., Billerica, MA). The degree of labeling, determined by mass spectrometry, is 1.6.

### Step 2): SPAAC (Strain-Promoted Azide-Alkyne Cycloaddition) Reaction with N3-PEG3-vc-PAB-MMAE:

The xiRB4_IgG1_DBCO-DOTAGA conjugate is then subjected to a click-chemistry reaction with 10 equivalents of N3-PEG3-vc-PAB-MMAE per DBCO (10mM in DMSO). The antibody concentration is subsequently adjusted to 5mg/ml by adding PBS, and the reaction medium is stirred at 37°C for 4 hours. The conjugate is then purified by protein A affinity chromatography (Hitrap Mabselect Xtra column, Cytiva) and the degree of labeling is determined by mass spectrometry: DOL (degree of labeling) = 1.5.

### 2. Results

### Characterization of anti-human ET_{B} specific monoclonal antibodies

As described previously, rendomab-B1 was initially selected from 24 antibodies obtained through genetic immunization based on its unique property to behave as a remarkably potent antagonist of human ET_{B} (Allard B. et al, 2013). However this mAb displayed a very low affinity for ET_{B} expressed on tumor cells, which prompted us to look for the potential presence, among the 23 other anti-ET_{B} mAbs that we produced, of antibodies that could recognize the tumor-associated form of human ET_{B}. Toward that end, the binding properties of all the mAbs on Chinese hamster ovary (CHO) cells stably transfected with human ET_{B} (CHO-ET_{B}) and different melanoma cell lines were investigated. As expected, all mAbs were able to bind CHO-ET_{B}, although with different intensities, since this cell line was used for the screening of anti-ET_{B} antibodies-expressing hybridomas. This is illustrated in Fig. 1A for 9 mAbs selected here for their high Bmax value on CHO- ET_{B} (rendomab B4 and 8 other rendomabs, BS1 to BS8); 3 mAbs belonged to the IgG2B subclass, 3 to the IgG1 and 3 to the IgG3 subclass. The affinities of these 9 mAbs for CHO- ET_{B} are shown in Fig. 1B, and, as can be observed, no correlation was found between the isotypic class of the mAbs, Bmax, and affinity.

Most interestingly, all 9 selected mAbs appeared to be able to bind ET_{B} expressed at the surface of the 3 melanoma cell lines tested: UACC-257, WM-266-4 and SLM8. Fig. 1C shows their apparent affinities for UACC-257, and a detailed analysis of rendomab-B4 binding on the 3 different melanoma cells is shown below. In addition, among the 23 new antibodies that we tested, some other mAbs were totally unable to bind ET_{B} expressed on any tumoral cell line, as was observed for the rendomab B1 that was already described (Allard B. et al, 2013) whereas others recognized ET_{B} expressed only on a given tumoral cell line (results not shown). Since, as illustrated in Fig. 1 B and C, rendomab B4 (Rmb B4) exhibited the highest apparent affinity values not only for CHO-ET_{B}, but above all for the tumoral UACC-257 cell line, it was selected for further characterization of its functional properties.

### Rendomab-B4 specifically recognizes with high affinity human ET_{B} overexpressed in CHO cell line

It is well established that the 3 ETs bind ET_{B} with comparable affinities. First, we checked the binding properties of ETs on CHO-ET_{B} by using fluorescent ET-1 and ET-3 (ET-1-FAM and ET-3-FAM, respectively). Flow cytometry experiments showed that the 2 labeled ETs bound to CHO-ET_{B} with the same Kd value ~ 1 nM, and the maximal binding was obtained at 100 nM for the 2 ligands (Fig. 2A and B). Data presented in Fig. 2C show that rendomab-B4 binding was dose-dependent, saturable, and reached a plateau for a 50 nM concentration.half maximal binding was about 0.15±0.03nM (mean ± s.d), reflecting the high affinity of rendomab-B4 for hET_{B}. Additional experiments show that rendo- mab-B4 bound neither to untransfected CHO cells that do not express ET_{B}, nor to a rat leiomyoma cell line (ELT-3) that endogenously expresses ET_{B} at high level (Raymond MN et al, 2009), and did not cross-react with ET_{A} expressed in CHO cells (data not shown). Altogether, these data indicated that rendomab-B4 was specific to human ET_{B}. This specific binding of rendomab-B4 to ET_{B} was visualized by immunofluorescence experiments showing that the mAb displayed a marked membrane labeling in CHO-ET_{B} cells, but not on control untransfected cells (Fig. 2D).

### Rendomab-B4 does not compete with ET-1 and ET-3 for binding on CHO-ET_{B}

Competition experiments were performed to characterize the interaction of rendomab-B4 with ET_{B} expressed in CHO in the presence of ET-1-FAM or ET-3-FAM. Data depicted in Fig. 3 show that ET-1 and ET-3 inhibited ET-1-FAM (Fig. 3A) and ET-3-FAM (Fig. 3B), respectively, with IC50 estimated at 0.17 ± 0.07 and 0.31 ±0.01 nM, respectively. The specific ET_{A} antagonist BQ123 failed to reduce ET-1-FAM and ET-3-FAM binding in CHO-ET_{B}. In contrast, BQ788, the specific antagonist for ET_{B}, inhibited ET-1-FAM and ET-3-FAM binding in a dose-dependent manner, with IC50 values around 25.2 ± 2.8 and 8.9 ± 4.0 nM, respectively. Rendomab-B4, and its corresponding isotype control mAb, failed to impair the binding of ET-1-FAM (Fig. 3A) and ET-3-FAM (Fig. 3B). Conversely, rendomab-B4 binding was not inhibited by ET-1, ET-3 or random control peptide (Fig. 3C). These data demonstrated that rendomab-B4 and ETs are not competing for receptor binding, suggesting that their binding sites on ET_{B} are distinct.

### Rendomab-B4 recognizes a discontinuous epitope on hET_{B} N-terminal domain

To further characterize the binding site of rendomab-B4 on hETs, epitope mapping experiments were performed. Pepscan membranes spotted with overlapping dodecapeptides, frame- shifted by one residue, and corresponding to the N-terminal part and extracellular loops (E1, E2, E3) of the hETs were used. The immunization protocol that was carried out was expected to lead to the production of antibodies directed against the extracellular parts of the ET_{B}.

The membrane incubated with rendomab-B4 displayed 2 immunoreactive regions, one from A21 to B8 and another from C17 to C24 spots, corresponding to ET_{B} residues 28 to 38 and 70 to 77, respectively. The two series of immunoreactive peptides are part of the N-terminal domain. The deduced minimal sequences were ERGFPPDRATP (SEQ ID NO:9) and EVPKGDRT (SEQ ID NO:10). The first sequence corresponds to the most N-terminal region of the mature receptor, once signal peptide is cleaved. The absence of any similarity between the 2 sequences suggested that rendomab- B4 recognized a conformational epitope formed by the juxtaposition of 2 regions of the receptor. These two sequences are unique to human ET_{B} and are not found in any other human proteins. Interestingly, the 2 sequences forming the epitope are not conserved between human and rodents, and each peptide differs by 3 amino acids. This may explain why rendomab-B4 is unable to bind ET_{B}-expressing rat cells, and suggest that these residues are crucial for rendomab-B4 binding.

To confirm this, human and rat ET_{B} were expressed in CHO cells and tested for their ability to bind rendomab-B4 in an in vitro immunoprecipitation assay. Because no anti-ET_{B} antibody that works well in western blot was available, human and rat ET_{B} were expressed as GFP fusion proteins and detected with an anti-GFP antibody. Human (hETs) and rat (rETa) GFP-fused ET_{B}, immunoprecipitated with an anti-GFP nanobody followed by protein gel blot analysis with another anti GFP antibody, appear as several immunoreactive bands. For each receptor, the band of higher apparent molecular mass is likely to represent the full-length GFP-tagged receptor as its mass is consistent with the calculated theoretical mass of the protein (about 75 kDa). The other bands of lower molecular mass may represent N-terminally truncated forms of the receptor since the receptors were immunoprecipitated through their C-termi- nal GFP fusion.

When hET_{B}-GFP was subjected to immunoprecipitation with rendomab-B4 followed by western blot analysis with an anti-GFP antibody, only one immunoreactive band was detected. This band corresponds to the higher mass band visible in the lower panel. This result shows that rendomab-B4 is able to interact and precipitate the full-length ET_{B}, but not its N-terminally truncated forms. This result is consistent with the finding of the epitope sequence in its N-terminal domain. When the same experiment was performed on rET_{B}-GFP instead of hET_{B}-GFP, no immunoreactive band was detected, confirming that rendomab-B4 does not cross-react with rET_{B} and strengthening the hypothesis that the amino acids that differ between human and rat ET_{B} are important for rendomab-B4 binding.

### Rendomab-B4 specifically recognizes ET_{B} in UACC-257 melanoma cell line: binding properties

Considering that rendomab-B4 was able to bind human ET_{B} in CHO cells with high affinity and specificity, its binding on human melanoma cell lines and on non-malignant cell lines expressing ET_{B} was further investigated. Results in Fig. 4A show that rendomab-B4 clearly displays saturable binding curves on UACC-257 cells. Binding curves obtained with WM- 266-4 and SLM8 also tend to saturate and could be easily fitted to sigmoid curves. Analysis of the binding curves gave half-maximal binding concentration values of 0.14 ± 0.03 nM, 1.5 ± 0.32 nM and ~10 nM for UACC-257, WM-266-4 and SLM8, respectively. In contrast, binding curves obtained with HEK293T and HUVEC clearly did not tend to saturate, and these curves could not be fitted to sigmoid curves, indicating that no specific binding of rendomab-B4 was observed in these non-tumoral, yet ETs-expressing cells (Fig. 4A). This result further supports our previous assumption of the occurrence of tumor-specific conformations of ET_{B}.25

Since its apparent affinity was also higher in UACC-257 than in the 2 other melanoma cell lines and, in addition, among the 9 selected mAbs, rendomab-B4 displayed the best apparent affinity for UACC-257 (Fig. 1C), this cell line was chosen for further characterization of the fine binding properties and pharmacological effects of the antibody on tumor cells. Fig. 4B shows the saturable binding on UACC-257 cells of labeled ET- 3, which is a specific agonist of ET_{B}, known to play a leading role in melanoma development. This binding is characterized by an EC50 value of 3.8 ± 0.1 nM, which is in the same order of magnitude as that obtained in CHO-ET_{B} cells (Fig. 2B). Competition experiments revealed that, as expected, ET-3 and BQ788 were able to fully inhibit ET-3-FAM binding, while control antibody and BQ123 were without any effect (Fig. 4C).

Moreover, as already observed in CHO-ET_{B} cells, rendomab- B4 was not able to reduce ET-3-FAM in UACC-257 cells, confirming that rendomab-B4 is not a competitor of ET-3 binding on hETs.

### Rendomab-B4 is internalized in UACC-257 cells

Given that ET_{B}-targeting-rendomab-B4 binds UACC-257 cells with high affinity, and that ET_{B} is known to have high internalization and turnover rates (Oksche A. et al, 2000; Bremnes T. et al, 2000), the internalization of the rendomab-B4 was studied. Internalization of a mAb is a crucial property because it allows uptake of a cytotoxic drug coupled to the mAb into cancer cells expressing the target.17,29 Cytometry experiments were carried out to study the internalization of ET_{B} in UACC-257 cells (Fig. 5A). Because no competition was observed between ET-3 and rendomab-B4 binding on UACC-257 (Fig. 4C), rendomab-B4 was used to quantify receptors present at the surface of the cells after incubation of living adherent cells with ET-3 in different conditions. The maximal binding was determined by incubating the cells with ET-3 for h at 4°C, a condition where internalization is blocked. Incubation of the cells with ET-3 for 1 h at 37°C, a temperature for which internalization can occur, led to a decrease of the fluorescent signal by about 70%. This signal decrease reflects a massive ET-3-mediated internalization of ET_{B}.

We also examined the internalization of the rendomab-B4 itself, and the results are shown in Fig. 5B. To avoid internalization of the receptor and to obtain the maximal binding, adherent UACC-257 cells were first incubated for 3 hours at 4°C with the rendomab-B4. To allow the internalization, the cells were incubated for 3 hours at 37°C in the presence of the antibody. This resulted in a signal decrease by about 40%. Moreover, when ET-3 was added to the cells and incubated for one additional hour at 37°C, still in the presence of rendomab-B4, the signal was further reduced by about 80%. In control experiments, no binding was observed with an isotypic control anti- body (data not shown). Thus, rendomab-B4 is internalized by UACC-257 cells, and this effect is increased in the presence of the agonist ET-3, reaching a percentage of internalization higher than that obtained with ET-3 alone, suggesting that rendomab-B4 by itself is able to promote ET_{B} endocytosis to some extent. Immunofluorescence analysis of rendomab-B4 binding on UACC-257 living cells at 4°C is shown in Fig. 5C.

The result clearly shows that rendomab-B4 bound to the UACC-257 cell surface. After 2 hours of incubation at 37°C, the labeling disappeared from the cell membrane (Fig. 5D) and appeared as a punctuate labeling in the cytoplasm, reflecting the internalization of the mAb. As expected, this labeling obtained with rendomab-B4 mainly colocalized with Early Endosome Antigen 1 (EEA1) staining (Fig. 5E and F), indicating that after 2 h ET-3 treatment, a large proportion of rendomab-B4 is located in early endosomes.

### Rendomab-B4 inhibits PLC but not ERK response due to ET_{B} receptor activation in UACC

Although rendomab-B4 does not compete with ET binding on hETs, we cannot exclude the possibility that it might exert allosteric modulation on receptor activity or might reduce the number of receptors at the cell surface, as suggested by the internalization results described above. Therefore, the effect of rendomab-B4 on signaling pathways coupled to hETs in UACC-257 cells was investigated. It is well established that ET_{B} is coupled to Gq and/or Gi families of G proteins, leading to the activation of phospholipase C (PLC) and ERK1/2 MAP kinases pathways. Data shown in Fig. 6A showed that incubation of UACC-257 cells with ET-1, but also with ET-3, stimulated the PLC activity by about 5 times, as reflected by the increased production of inositol phosphates (InsPs). Incubation of the cells for 2 hours in the presence of control isotype anti- body had no effect. In contrast, a similar treatment with the rendomab-B4 strongly reduced (by about 75%) the production of InsPs stimulated both by ET-1 and ET-3. These data indicate that rendomab-B4 is able to reduce the PLC transduction pathway without interfering with the binding of endogenous receptor ligands.

The MAP kinases pathway is known to be involved in tumor progression in melanoma. Therefore, we also investigated the potential effect of rendomab-B4 on this pathway. Our data confirmed that in UACC-257, both ET-1 and ET-3 increased ERK1/2 phosphorylation and activation, as previously shown (Asundi J. et al, 2011).

Indeed, in UACC-257, the 2 ETs induced a rapid increase of ERK1/2 phosphorylation, with a maximal response obtained at 5 to 10 min, followed by a progressive decrease (data not shown). The effect of rendomab-B4 was then tested on the activation of ERK1/2 by ET-1 and ET-3 (Fig. 6B and C). Data depicted in Fig. 6C clearly indicate that the activation of ERK1/2 was unaffected when UACC-257 were treated in the presence of rendomab-B4, as it was observed using control mAb. These results show that rendomab-B4 exhibits differential inhibitory effects on PLC and ERK pathways activated by ETs.

### Rendomab-B4 inhibits melanoma cell migration induced by endothelin

It is now well established that the ET axis plays an important role in cancer progression, triggering cellular events involved in cancer cells invasion. Given this crucial role, we next studied the effect of rendomab-B4 on ET-1-induced migration of UACC-257. Data shown in Fig. 7 demonstrated that the migration of the melanoma cell line was increased by 3-fold in the presence of ET-1. Incubation of the cells with control antibody failed to modify the cell migration due to ET-1. By contrast, rendomab-B4 completely abolished UACC-257 cells migration induced by ET-1. We verified that under similar conditions, the viability and the number of cells were not affected by ET-1 and rendomab-B4 (data not shown). Altogether, these results show that, although rendomab-B4 does not prevent ET binding on ET_{B}, it is able to inhibit several of its effects on melanoma cells.

### BIBLIOGRAPHIC REFERENCES

Adumeau P et al., 2018 A Site-Specifically Labeled Antibody-Drug Conjugate for Simultaneous Therapy and ImmunoPET. Mol Pharm. 2018 March 05; 15(3): 892-898.
Adumeau P. et al., 2022. Site-Specific, Platform-Based Conjugation Strategy for the Synthesis of Dual-Labeled Immunoconjugates for Bimodal PET/NIRF Imaging of HER2-Positive Tumors Bioconjugate Chem. 2022, 33, 530-540
Allard B, Priam F, Deshayes F, Ducancel F, Boquet D, Wijkhuisen A, Couraud JY. Electroporationaided DNA immunization generates polyclonal antibodies against the native conformation of human endothelin B receptor. DNA Cell Biol 2011; 30:727-37.
Allard B, Wijkhuisen A, Borrull A, Deshayes F, Priam F, Lamourette P, Ducancel F, Boquet D, Couraud JY. Generation and characterization of rendomab-B1, a monoclonal antibody displaying potent and specific antagonism of the human endothelin B receptor. MAbs 2013; 5:56-69.
Asundi J, Lacap JA, Clark S, Nannini M, Roth L, Polakis P. MAPK pathway inhibition enhances the efficacy of an anti-endothelin B receptor drug conjugate by inducing target expression in melanoma. Mol Cancer Ther 2014; 13:1599-610.
Asundi J, Reed C, Area J, McCutcheon K, Ferrando R, Clark S, Luis E, Tien J, Firestein R, Polakis P. An antibody-drug conjugate targeting the endothelin B receptor for the treatment of melanoma. Clin Cancer Res 2011; 17:965-75.
Berger Y, Bernasconi CC, Juillerat-Jeanneret L. Targeting the endothelin axis in human melanoma: combination of endothelin receptor antagonism and alkylating agents. Exp Biol Med (Maywood) 2006; 231:1111-9.
Bremnes T, Paasche JD, Mehlum A, Sandberg C, Bremnes B, Attramadal H. Regulation and intracellular trafficking pathways of the endothelin receptors. J Biol Chem 2000; 275:17596-604.
Costoplus et al. 2019 (Peptide-Cleavable Self-immolative Maytansinoid Antibody-Drug Conjugates Designed To Provide Improved Bystander Killing. ACS Med Chem Lett. 2019 Sep 27;10(10):1393-1399),
Dickgiesser et al. 2020 (Site-Specific Conjugation of Native Antibodies Using Engineered Microbial Transglutaminases. Bioconjug Chem. 2020 Mar 12. doi: 10.1021/acs.bioconjchem.0c00061).
Jeger S. et al., 2010, Site-specific and stoichiometric modification of antibodies by bacterial transglutaminase. Angew. Chem. Int. Ed., 49, 9995-9997
Kefford RF, Clingan PR, Brady B, Ballmer A, Morganti A, Hersey P. A randomized, double-blind, placebo-controlled study of high-dose bosentan in patients with stage IV metastatic melanoma receiving first-line dacarbazine chemotherapy. Mol Cancer 2010; 9:69.
Kondoh et al, 1990 ("Isolation of anti-endothelin receptor monoclonal antibodies for use in receptor characterization", BBRC, vol. 172, pages 503-510
Lahav R. Endothelin receptor B is required for the expansion of melanocyte precursors and malignant melanoma. Int J Dev Biol 2005; 49:173-80.
Laune D, Molina F, Ferrieres G, Villard S, Bes C, Rieunier F, Chardes T, Granier C. Application of the Spot method to the identification of peptides and amino acids from the antibody paratope that contribute to antigen binding. J Immunol Methods 2002; 267:53-70.
Lefranc, M.-P.,et al., Dev. Comp. Immunol., 27, 55-77 (2003)
Maguire JJ, Davenport AP. Endothelin receptors and their antagonists. Semin Nephrol 2015; 35:125-36.
Oksche A, Boese G, Horstmeyer A, Papsdorf G, Furkert J, Beyermann M, Bienert M, Rosenthal W. Evidence for downregulation of the endothelin-B-receptor by the use of fluorescent endothelin-1 and a fusion protein consisting of the endothelin-B-receptor and the green fluorescent protein. J Cardiovasc Pharmacol 2000; 36:S44-7.
Proietti I. et al. BRAF Inhibitors: Molecular Targeting and Immunomodulatory Actions. Cancers 2020, 12(7), 1823.
Raymond MN, Robin P, De Zen F, Vilain G, Tanfin Z. Differential endothelin receptor expression and function in rat myometrial cells and leiomyoma ELT3 cells. Endocrinology 2009; 150:4766-76.
Recondo G, Diaz Canton E, de la Vega M, Greco M, Valsecchi ME. Therapeutic options for HER-2 positive breast cancer: Perspectives and future directions. World J Clin Oncol 2014; 5:440-54.
Rosanò, L., Spinella, F., Bagnato, A., 2013. Endothelin 1 in cancer: biological implications and therapeutic opportunities. Nat Rev Cancer 13, 637-651.
Russo A, Ficili B, Candido S, Pezzino FM, Guarneri C, Biondi A, Travali S, McCubrey JA, Spandidos DA, Libra M. Emerging targeted therapies for melanoma treatment (review). Int J Oncol 2014; 45:516-24.
Shihoya, W., Nishizawa, T., Okuta, A., Tani, K., Dohmae, N., Fujiyoshi, Y., Nureki, O., Doi, T., 2016. Activation mechanism of endothelin ETB receptor by endothelin-1. Nature 537, 363.
Sonzini et al. 2020 (Improved Physical Stability of an Antibody-Drug Conjugate Using Host-Guest Chemistry. Bioconjug Chem. 2020 Jan 15;31(1):123-129),
Strop P. Versatility of Microbial transglutaminase, Bioconjugate Chem. 2014, 25, 855-862
Topalian SL, Sznol M, McDermott DF, Kluger HM, Carvajal RD, Sharfman WH, Brahmer JR, Lawrence DP, Atkins MB, Powderly JD, et al. Survival, durable tumor remission, and long-term safety in patients with advanced melanoma receiving nivolumab. J Clin Oncol 2014; 32:1020-30.
Turneh PC, Harview CL, Yearley JH, Shintaku IP, Taylor EJ, Robert L, Chmielowski B, Spasic M, Henry G, Ciobanu V, et al. PD-1 blockade induces responses by inhibiting adaptive immune resistance. Nature 2014; 515:568-71.
Weathers, S.-P., Rood-Breithaupt, J., de Groot, J., Thomas, G., Manfrini, M., Penas-Prado, M., Puduvalli, V.K., Zwingelstein, C., Yung, W.K.A., 2021. Results of a phase I trial to assess the safety of macitentan in combination with temozolomide for the treatment of recurrent glioblastoma. Neurooncol Adv 3, vdab141.
Wouters, J., Hunger, R.E., Garrod, T., Dubuis, B., Hunziker, T., Oord, J.J. van den, Coutre, R.L., 2015. First-in-Human Proof-of-Concept Study: Intralesional Administration of BQ788, an Endothelin Receptor B Antagonist, to Melanoma Skin Metastases. The Oncologist 20, 1121-1122.
Yamaguchi et al, 2004 ("Characterization and application of monoclonal antibodies against human endothelin B receptor expressed in insect cells", Biotechnology Letters, vol. 26, pages 293-299)

## Claims

1. An antibody conjugate comprising:
i) an antibody variable domain recognizing specifically the human endothelin receptor subtype B (ET_{B}), said variable domain comprising the CDRs of SEQ ID NO:1-6, or CDRs having a sequence of at least 90%, 95% and 98% identity with sequences SEQ ID NO:1-6 after optimal alignment,
ii) at least one trivalent linking platform able to bind two kinds of molecules chosen from cytotoxic and detection molecules, said platform being covalently coupled to the antibody part of the antibody conjugate.

2. The antibody conjugate of claim 1, comprising:
- The light and heavy chain variable domains of the antibody produced by the hybridoma deposited at the CNCM (Collection Nationale de Cultures de Microorganismes, Institut Pasteur, 25 rue du Docteur Roux, F-75724 PARIS CEDEX 15) on 14 March 2023 under the accession number CNCM I-5937,
- human light and heavy chain constant domains.

3. The antibody conjugate of claim 1 or 2, comprising:
- the light chain variable domain (V_{L}) of sequence SEQ ID NO: 7 or an amino acid sequence having at least 80%, preferably 85%, 90%, 95% and 98% identity with SEQ ID NO: 7 after optimal alignment, and a human Ig Kappa or lambda constant domain,
- the heavy chain variable domain (V_{H}) of sequence SEQ ID NO: 8 or an amino acid sequence having at least 80%, preferably 85%, 90%, 95% and 98% identity with SEQ ID NO: 8 after optimal alignment, and the CH1, CH2 and CH3 domains of a human IgG, IgM, IgE, IgD or IgA antibody.

4. The antibody conjugate of any of claims 1 to 3, comprising:
- the light chain variable domain (V_{L}) of sequence SEQ ID NO: 7 and a human Ig Kappa constant domain, eg., said light chain having e.g. the sequence SEQ ID NO:22,
- the heavy chain variable domain (V_{H}) of sequence SEQ ID NO: 8 and the CH1, CH2, CH3 and hinge domains of the human IgG1 antibody, e.g., said heavy chain having e.g. the sequence SEQ ID NO:23.

5. The antibody conjugate of any of claims 1-4, comprising a functional fragment of the antibody produced by the hybridoma deposited at the CNCM (Collection Nationale de Cultures de Microorganismes, Institut Pasteur, 25 rue du Docteur Roux, F-75724 PARIS CEDEX 15) on 14 March 2023 under the accession number CNCM I-5937, or of the humanized or fully human version thereof, said fragment being preferably a Fv, Fab, (Fab')₂, Fab', scFv, scFv-Fc fragment or diabody of said antibody.

6. The antibody conjugate of claim 5, comprising a Fab functional fragment comprising:
- the light chain variable domain (V_{L}) of sequence SEQ ID NO: 7 and a human Ig Kappa constant domain, said light chain having e.g. the sequence SEQ ID NO:24,
- the heavy chain variable domain (V_{H}) of sequence SEQ ID NO: 8, the CH1 domain of the human IgG1 antibody and the human IgG1 hinge, said heavy chain having e.g. the sequence SEQ ID NO:25.

7. The antibody conjugate of claim 56, comprising a Fab functional fragment linked to a Q tag having for example the sequence LLQGA (SEQ ID NO:27).

8. The antibody conjugate according to any one of claims 1 to 7, wherein each chain of its antibody part is covalently coupled to one or two cytotoxic molecules, one or two detection molecules or to one cytotoxic molecule and one detection molecule.

9. The antibody conjugate according to any one of claims 1 to 8, wherein its antibody part is covalently coupled to MMAE and Lu-177.

10. The antibody conjugate according to any one of claims 1 to 9, wherein the cytotoxic and/or detection molecules are covalently bound to a Q amino acid residue present in the antibody polypeptide, by means of a trivalent linking platform.

11. The antibody conjugate according to any one of claim 10, wherein the trivalent linking platform is a lysine trivalent moiety, which is preferably coupled to:
- either one or two chelating agent(s) chosen in the group consisting of: 1,4,7-triazacyclononane-1,4,7-triacetic acid (NOTA), dodecane tetracetic acid (DOTA), 1,4,7, 10-tetrakis(carbamoylmethyl)-1 ,4, 7, 10-tetracyclododecane (TCMC), 1-N-(4-aminobenzyl)-3,6,10,13,16,19-hexazabicyclo[6.6.6]eicosane-1,8-diamine (DiAmSar), N,N-bis(2- hydroxybenzyl)ethylenediamine-N,N-diacetic acid (HBED), deferoxamine (DFO), and diethylenetraminepentacetic acid (DTPA), N,N'-bis[(6-carboxy-2-pyridil)methyl]- 4,13-diaza-18crown-6 (MACROPA), 2,2',2"-nitrilotriacetic acid (NTA), 2,2- bis(hydroxymethyl)-2,2',2"-nitrilotriethanol (BisTris), ethylene glycol-bis(2-aminoethyl ether)-N,N,N',N'-tetraacetic acid (EGTA), ethylenediamine-N,N,N',N'-tetraacetic acid (EDTA), 1,2-bis(2-aminophenoxy)ethane-N,N,N',N'-tetraacetic acid (BAPTA), 1,4,7,10- tetraazacyclododecane-1,7-diacetic acid (DO2A), 1,4,7,10-tetraazacyclododecane-1, 4,7- triacetic acid (DO3A), desferrioxamine B, and their derivatives, including DOTAGA and (R)-NODAGA,
and/or
- either one or two drug(s),
and/or
- either one or two chemical coupling agent(s) chosen in the group consisting of: DBCO (dibenzylcyclooctyne), DIBAC (diethylaminocyclooctine, or BARAC), TCO (trans-cyclooctene), BCN (bicyclo[6.1.0]nonyne) and tetrazine derivatives.

12. The antibody conjugate according to any one of claims 1 to 11, containing:
- DOTAGA and MMAE that are conjugated by means of a lysine trivalent moiety, or
- MMAE and DFO that are conjugated by means of a lysine trivalent moiety.

13. A pharmaceutical composition comprising the antibody conjugate according to any one of claims 1 to 12, and a pharmaceutically-acceptable carrier, preferably for use as a medicament, more preferably for use for treating melanoma and other solid cancers in which the human endothelin B receptor is overexpressed, such as glioblastoma, pancreatic cancer, ovarian cancer, bladder cancer, lung cancer, kidney cancer, vulvar cancer or liquid cancers in which the human endothelin B receptor is overexpressed as leukemia, lymphoma, and myeloma..

14. Use of the antibody conjugate according to any one of claims 1-12, wherein said antibody conjugate comprises at least one detection molecule, preferably at least two different detection molecules, as a diagnostic agent.

15. Use of the antibody conjugate according to any one of claims 1-12, wherein said antibody conjugate comprises at least one detection molecule, preferably at least two detection molecules, in:
a) a method of diagnosing a tumor by imaging,
b) a surgical treatment method involving tumor resection guided by fluorescence or an isotopic imaging probe,
c) a method of monitoring the effectiveness of an antitumor treatment by imaging, or
d) any combination of methods a), b), and c).
